# EUROPEAN PATENT APPLICATION

(11) **EP 1 394 268 A1**
(43) Date of publication of application: **03.03.2004**
(21) Application number: 02292054.0
(22) Date of filing: 16.08.2002
(51) Int. Cl.: C12Q 1/68

(54) **HNF1alpha as a tumor suppressor gene, and diagnostic and therapeutic applications thereof**

(71) Applicant: INSTITUT NATIONAL DE LA SANTE ET DE LA RECHERCHE MEDICALE (INSERM), 75013 Paris (FR)
(72) Inventor: Zucman-Rossi, Jessica, 75014 Paris (FR); Laurent-Puig, Pierre, 92190 Meudon (FR); Bluteau, Olivier, 75012 Paris (FR); Bioulac-Sage, Paulette, 33200 Bordeaux (FR); Jeannot, Emmanuelle, 75005 Paris (FR); Marques, Juan Martin, Montevideo (UY); Balabaud, Charles, 33000 Bordeaux (FR)
(74) Representative: Bernasconi, Jean Raymond

(57) **Abstract**

The present invention relates to diagnostic methods and therapeutics in the field of tumors involving Hepatocyte Nuclear Factor 1α (HNF1α). The invention provides an *in vitro* method for the diagnosis of a tumor in a patient, which method comprises determining the expression or activity of HNF1α. The invention also provides a HNF1α gene therapy and protein therapy method.

SEQUENCE LISTING

<110> INSERM

<120> HNF1alpha as a tumor suppressor gene, and diagnostic
      and therapeutic applications thereof

<130> BEt 02/0778

<140>
<141>

<160> 27

<170> PatentIn Ver. 2.1

<210> 1
<211> 1896
<212> DNA
<213> Homo sapiens

<220>
<221> CDS
<222> (1)..(1896)

<400> 1
atg gtt tct aaa ctg agc cag ctg cag acg gag ctc ctg gcg gcc ctg    48
Met Val Ser Lys Leu Ser Gln Leu Gln Thr Glu Leu Leu Ala Ala Leu
  1               5                  10                  15

ctc gag tca ggg ctg agc aaa gag gca ctg atc cag gca ctg ggt gag    96
Leu Glu Ser Gly Leu Ser Lys Glu Ala Leu Ile Gln Ala Leu Gly Glu
             20                  25                  30

ccg ggg ccc tac ctc ctg gct gga gaa ggc ccc ctg gac aag ggg gag    144
Pro Gly Pro Tyr Leu Leu Ala Gly Glu Gly Pro Leu Asp Lys Gly Glu
         35                  40                  45

tcc tgc ggc ggc ggt cga ggg gag ctg gct gag ctg ccc aat ggg ctg    192
Ser Cys Gly Gly Gly Arg Gly Glu Leu Ala Glu Leu Pro Asn Gly Leu
     50                  55                  60

ggg gag act cgg ggc tcc gag gac gag acg gac gac gat ggg gaa gac    240
Gly Glu Thr Arg Gly Ser Glu Asp Glu Thr Asp Asp Asp Gly Glu Asp
 65                  70                  75                  80

ttc acg cca ccc atc ctc aaa gag ctg gag aac ctc agc cct gag gag    288
Phe Thr Pro Pro Ile Leu Lys Glu Leu Glu Asn Leu Ser Pro Glu Glu
                 85                  90                  95

gcg gcc cac cag aaa gcc gtg gtg gag acc ctt ctg cag gag gac ccg    336
Ala Ala His Gln Lys Ala Val Val Glu Thr Leu Leu Gln Glu Asp Pro
            100                 105                 110

tgg cgt gtg gcg aag atg gtc aag tcc tac ctg cag cag cac aac atc    384
Trp Arg Val Ala Lys Met Val Lys Ser Tyr Leu Gln Gln His Asn Ile
        115                 120                 125

cca cag cgg gag gtg gtc gat acc act ggc ctc aac cag tcc cac ctg    432
Pro Gln Arg Glu Val Val Asp Thr Thr Gly Leu Asn Gln Ser His Leu
    130                 135                 140

tcc caa cac ctc aac aag ggc act ccc atg aag acg cag aag cgg gcc    480
Ser Gln His Leu Asn Lys Gly Thr Pro Met Lys Thr Gln Lys Arg Ala
145                 150                 155                 160

gcc ctg tac acc tgg tac gtc cgc aag cag cga gag gtg gcg cag cag    528
Ala Leu Tyr Thr Trp Tyr Val Arg Lys Gln Arg Glu Val Ala Gln Gln
                165                 170                 175

ttc acc cat gca ggg cag gga ggg ctg att gaa gag ccc aca ggt gat    576
Phe Thr His Ala Gly Gln Gly Gly Leu Ile Glu Glu Pro Thr Gly Asp
            180                 185                 190

gag cta cca acc aag aag ggg cgg agg aac cgt ttc aag tgg ggc cca    624
Glu Leu Pro Thr Lys Lys Gly Arg Arg Asn Arg Phe Lys Trp Gly Pro
        195                 200                 205

gca tcc cag cag atc ctg ttc cag gcc tat gag agg cag aag aac cct    672
Ala Ser Gln Gln Ile Leu Phe Gln Ala Tyr Glu Arg Gln Lys Asn Pro
    210                 215                 220

agc aag gag gag cga gag acg cta gtg gag gag tgc aat agg gcg gaa    720
Ser Lys Glu Glu Arg Glu Thr Leu Val Glu Glu Cys Asn Arg Ala Glu
225                 230                 235                 240

tgc atc cag aga ggg gtg tcc cca tca cag gca cag ggg ctg ggc tcc    768
Cys Ile Gln Arg Gly Val Ser Pro Ser Gln Ala Gln Gly Leu Gly Ser
                245                 250                 255

aac ctc gtc acg gag gtg cgt gtc tac aac tgg ttt gcc aac cgg cgc    816
Asn Leu Val Thr Glu Val Arg Val Tyr Asn Trp Phe Ala Asn Arg Arg
            260                 265                 270

aaa gaa gaa gcc ttc cgg cac aag ctg gcc atg gac acg tac agc ggg    864
Lys Glu Glu Ala Phe Arg His Lys Leu Ala Met Asp Thr Tyr Ser Gly
        275                 280                 285

ccc ccc cca ggg cca ggc ccg gga cct gcg ctg ccc gct cac agc tcc    912
Pro Pro Pro Gly Pro Gly Pro Gly Pro Ala Leu Pro Ala His Ser Ser
    290                 295                 300

cct ggc ctg cct cca cct gcc ctc tcc ccc agt aag gtc cac ggt gtg    960
Pro Gly Leu Pro Pro Pro Ala Leu Ser Pro Ser Lys Val His Gly Val
305                 310                 315                 320

cgc tat gga cag cct gcg acc agt gag act gca gaa gta ccc tca agc    1008
Arg Tyr Gly Gln Pro Ala Thr Ser Glu Thr Ala Glu Val Pro Ser Ser
                325                 330                 335

agc ggc ggt ccc tta gtg aca gtg tct aca ccc ctc cac caa gtg tcc    1056
Ser Gly Gly Pro Leu Val Thr Val Ser Thr Pro Leu His Gln Val Ser
            340                 345                 350

ccc acg ggc ctg gag ccc agc cac agc ctg ctg agt aca gaa gcc aag    1104
Pro Thr Gly Leu Glu Pro Ser His Ser Leu Leu Ser Thr Glu Ala Lys
        355                 360                 365

ctg gtc tca gca gct ggg ggc ccc ctc ccc cct gtc agc acc ctg aca    1152
Leu Val Ser Ala Ala Gly Gly Pro Leu Pro Pro Val Ser Thr Leu Thr
    370                 375                 380

gca ctg cac agc ttg gag cag aca tcc cca ggc ctc aac cag cag ccc     1200
Ala Leu His Ser Leu Glu Gln Thr Ser Pro Gly Leu Asn Gln Gln Pro
385                 390                 395                 400

cag aac ctc atc atg gcc tca ctt cct ggg gtc atg acc atc ggg cct     1248
Gln Asn Leu Ile Met Ala Ser Leu Pro Gly Val Met Thr Ile Gly Pro
                405                 410                 415

ggt gag cct gcc tcc ctg ggt cct acg ttc acc aac aca ggt gcc tcc     1296
Gly Glu Pro Ala Ser Leu Gly Pro Thr Phe Thr Asn Thr Gly Ala Ser
            420                 425                 430

acc ctg gtc atc ggc ctg gcc tcc acg cag gca cag agt gtg ccg gtc     1344
Thr Leu Val Ile Gly Leu Ala Ser Thr Gln Ala Gln Ser Val Pro Val
        435                 440                 445

atc aac agc atg ggc agc agc ctg acc acc ctg cag ccc gtc cag ttc     1392
Ile Asn Ser Met Gly Ser Ser Leu Thr Thr Leu Gln Pro Val Gln Phe
    450                 455                 460

tcc cag ccg ctg cac ccc tcc tac cag cag ccg ctc atg cca cct gtg     1440
Ser Gln Pro Leu His Pro Ser Tyr Gln Gln Pro Leu Met Pro Pro Val
465                 470                 475                 480

cag agc cat gtg acc cag aac ccc ttc atg gcc acc atg gct cag ctg     1488
Gln Ser His Val Thr Gln Asn Pro Phe Met Ala Thr Met Ala Gln Leu
                485                 490                 495

cag agc ccc cac gcc ctc tac agc cac aag ccc gag gtg gcc cag tac     1536
Gln Ser Pro His Ala Leu Tyr Ser His Lys Pro Glu Val Ala Gln Tyr
            500                 505                 510

acc cac acg ggc ctg ctc ccg cag act atg ctc atc acc gac acc acc     1584
Thr His Thr Gly Leu Leu Pro Gln Thr Met Leu Ile Thr Asp Thr Thr
        515                 520                 525

aac ctg agc gcc ctg gcc agc ctc acg ccc acc aag cag gtc ttc acc     1632
Asn Leu Ser Ala Leu Ala Ser Leu Thr Pro Thr Lys Gln Val Phe Thr
    530                 535                 540

tca gac act gag gcc tcc agt gag tcc ggg ctt cac acg ccg gca tct     1680
Ser Asp Thr Glu Ala Ser Ser Glu Ser Gly Leu His Thr Pro Ala Ser
545                 550                 555                 560

cag gcc acc acc ctc cac gtc ccc agc cag gac cct gcc ggc atc cag     1728
Gln Ala Thr Thr Leu His Val Pro Ser Gln Asp Pro Ala Gly Ile Gln
                565                 570                 575

cac ctg cag ccg gcc cac cgg ctc agc gcc agc ccc aca gtg tcc tcc     1776
His Leu Gln Pro Ala His Arg Leu Ser Ala Ser Pro Thr Val Ser Ser
            580                 585                 590

agc agc ctg gtg ctg tac cag agc tca gac tcc agc aat ggc cag agc     1824
Ser Ser Leu Val Leu Tyr Gln Ser Ser Asp Ser Ser Asn Gly Gln Ser
        595                 600                 605

cac ctg ctg cca tcc aac cac agc gtc atc gag acc ttc atc tcc acc     1872
His Leu Leu Pro Ser Asn His Ser Val Ile Glu Thr Phe Ile Ser Thr
    610                 615                 620

cag atg gcc tct tcc tcc cag taa                                     1896

Gln Met Ala Ser Ser Ser Gln
625                 630

<210> 2
<211> 631
<212> PRT
<213> Homo sapiens

<400> 2
Met Val Ser Lys Leu Ser Gln Leu Gln Thr Glu Leu Leu Ala Ala Leu
1               5                   10                  15
Leu Glu Ser Gly Leu Ser Lys Glu Ala Leu Ile Gln Ala Leu Gly Glu
            20                  25                  30
Pro Gly Pro Tyr Leu Leu Ala Gly Glu Gly Pro Leu Asp Lys Gly Glu
        35                  40                  45
Ser Cys Gly Gly Gly Arg Gly Glu Leu Ala Glu Leu Pro Asn Gly Leu
    50                  55                  60
Gly Glu Thr Arg Gly Ser Glu Asp Glu Thr Asp Asp Asp Gly Glu Asp
65                  70                  75                  80
Phe Thr Pro Pro Ile Leu Lys Glu Leu Glu Asn Leu Ser Pro Glu Glu
                85                  90                  95
Ala Ala His Gln Lys Ala Val Val Glu Thr Leu Leu Gln Glu Asp Pro
            100                 105                 110
Trp Arg Val Ala Lys Met Val Lys Ser Tyr Leu Gln Gln His Asn Ile
        115                 120                 125
Pro Gln Arg Glu Val Val Asp Thr Thr Gly Leu Asn Gln Ser His Leu
    130                 135                 140
Ser Gln His Leu Asn Lys Gly Thr Pro Met Lys Thr Gln Lys Arg Ala
145                 150                 155                 160
Ala Leu Tyr Thr Trp Tyr Val Arg Lys Gln Arg Glu Val Ala Gln Gln
                165                 170                 175
Phe Thr His Ala Gly Gln Gly Gly Leu Ile Glu Glu Pro Thr Gly Asp
            180                 185                 190
Glu Leu Pro Thr Lys Lys Gly Arg Arg Asn Arg Phe Lys Trp Gly Pro
        195                 200                 205
Ala Ser Gln Gln Ile Leu Phe Gln Ala Tyr Glu Arg Gln Lys Asn Pro
    210                 215                 220
Ser Lys Glu Glu Arg Glu Thr Leu Val Glu Glu Cys Asn Arg Ala Glu
225                 230                 235                 240
Cys Ile Gln Arg Gly Val Ser Pro Ser Gln Ala Gln Gly Leu Gly Ser
                245                 250                 255
Asn Leu Val Thr Glu Val Arg Val Tyr Asn Trp Phe Ala Asn Arg Arg
            260                 265                 270
Lys Glu Glu Ala Phe Arg His Lys Leu Ala Met Asp Thr Tyr Ser Gly
        275                 280                 285
Pro Pro Pro Gly Pro Gly Pro Gly Pro Ala Leu Pro Ala His Ser Ser
    290                 295                 300
Pro Gly Leu Pro Pro Pro Ala Leu Ser Pro Ser Lys Val His Gly Val
305                 310                 315                 320
Arg Tyr Gly Gln Pro Ala Thr Ser Glu Thr Ala Glu Val Pro Ser Ser
                325                 330                 335
Ser Gly Gly Pro Leu Val Thr Val Ser Thr Pro Leu His Gln Val Ser
            340                 345                 350
Pro Thr Gly Leu Glu Pro Ser His Ser Leu Leu Ser Thr Glu Ala Lys
        355                 360                 365
Leu Val Ser Ala Ala Gly Gly Pro Leu Pro Pro Val Ser Thr Leu Thr
    370                 375                 380
Ala Leu His Ser Leu Glu Gln Thr Ser Pro Gly Leu Asn Gln Gln Pro
385                 390                 395                 400
Gln Asn Leu Ile Met Ala Ser Leu Pro Gly Val Met Thr Ile Gly Pro

                405                 410                 415
Gly Glu Pro Ala Ser Leu Gly Pro Thr Phe Thr Asn Thr Gly Ala Ser
            420                 425                 430
Thr Leu Val Ile Gly Leu Ala Ser Thr Gln Ala Gln Ser Val Pro Val
        435                 440                 445
Ile Asn Ser Met Gly Ser Ser Leu Thr Thr Leu Gln Pro Val Gln Phe
    450                 455                 460
Ser Gln Pro Leu His Pro Ser Tyr Gln Gln Pro Leu Met Pro Pro Val
465                 470                 475                 480
Gln Ser His Val Thr Gln Asn Pro Phe Met Ala Thr Met Ala Gln Leu
                485                 490                 495
Gln Ser Pro His Ala Leu Tyr Ser His Lys Pro Glu Val Ala Gln Tyr
            500                 505                 510
Thr His Thr Gly Leu Leu Pro Gln Thr Met Leu Ile Thr Asp Thr Thr
        515                 520                 525
Asn Leu Ser Ala Leu Ala Ser Leu Thr Pro Thr Lys Gln Val Phe Thr
    530                 535                 540
Ser Asp Thr Glu Ala Ser Ser Glu Ser Gly Leu His Thr Pro Ala Ser
545                 550                 555                 560
Gln Ala Thr Thr Leu His Val Pro Ser Gln Asp Pro Ala Gly Ile Gln
                565                 570                 575
His Leu Gln Pro Ala His Arg Leu Ser Ala Ser Pro Thr Val Ser Ser
            580                 585                 590
Ser Ser Leu Val Leu Tyr Gln Ser Ser Asp Ser Ser Asn Gly Gln Ser
        595                 600                 605
His Leu Leu Pro Ser Asn His Ser Val Ile Glu Thr Phe Ile Ser Thr
    610                 615                 620
Gln Met Ala Ser Ser Ser Gln
625                 630

<210> 3
<211> 20
<212> DNA
<213> Artificial Sequence

<220>
<223> Description of Artificial Sequence:PCR primer

<400> 3
tagctgtaag ctcctctggt 20

<210> 4
<211> 20
<212> DNA
<213> Artificial Sequence

<220>
<223> Description of Artificial Sequence:PCR primer

<400> 4
tacaggacct agagtcacct 20

<210> 5
<211> 18
<212> DNA
<213> Artificial Sequence

<220>
<223> Description of Artificial Sequence:PCR primer

<400> 5
gccagctgca gacggagc                                                    18


<210> 6
<211> 19
<212> DNA
<213> Artificial Sequence

<220>
<223> Description of Artificial Sequence:PCR primer

<400> 6
ggctcaccag gcccgatgg                                                   19


<210> 7
<211> 18
<212> DNA
<213> Artificial Sequence

<220>
<223> Description of Artificial Sequence:PCR primer

<400> 7
ccagtcccac ctgtccca                                                    18


<210> 8
<211> 19
<212> DNA
<213> Artificial Sequence

<220>
<223> Description of Artificial Sequence:PCR primer

<400> 8
ctgggaggaa gaggccatc                                                   19


<210> 9
<211> 18
<212> DNA
<213> Artificial Sequence

<220>
<223> Description of Artificial Sequence:PCR primer

<400> 9
gcagtgggtg caaggagt                                                    18


<210> 10
<211> 19
<212> DNA
<213> Artificial Sequence

<220>

<223> Description of Artificial Sequence:PCR primer

<400> 10
ctcagaaggg gggctcgtt                                                    19


<210> 11
<211> 19
<212> DNA
<213> Artificial Sequence

<220>
<223> Description of Artificial Sequence:PCR primer

<400> 11
tatggggaga gacagccct                                                    19


<210> 12
<211> 20
<212> DNA
<213> Artificial Sequence

<220>
<223> Description of Artificial Sequence:PCR primer

<400> 12
acctgtctgt gtaatgggga                                                   20


<210> 13
<211> 19
<212> DNA
<213> Artificial Sequence

<220>
<223> Description of Artificial Sequence:PCR primer

<400> 13
cctgagtacc cctagggac                                                    19


<210> 14
<211> 19
<212> DNA
<213> Artificial Sequence

<220>
<223> Description of Artificial Sequence:PCR primer

<400> 14
accccccaag caggcagta                                                    19


<210> 15
<211> 20
<212> DNA
<213> Artificial Sequence

<220>
<223> Description of Artificial Sequence:PCR primer

<400> 15
cccagatctg ccagcctcaa                                                    20

<210> 16
<211> 19
<212> DNA
<213> Artificial Sequence

<220>
<223> Description of Artificial Sequence:PCR primer

<400> 16
tgactgctgt cactgggac                                                     19

<210> 17
<211> 19
<212> DNA
<213> Artificial Sequence

<220>
<223> Description of Artificial Sequence:PCR primer

<400> 17
actagggctg tcaggcacg                                                     19

<210> 18
<211> 20
<212> DNA
<213> Artificial Sequence

<220>
<223> Description of Artificial Sequence:PCR primer

<400> 18
cagaaggggt gggaccaaca                                                    20

<210> 19
<211> 20
<212> DNA
<213> Artificial Sequence

<220>
<223> Description of Artificial Sequence:PCR primer

<400> 19
gagtttgaag tgctgagggc                                                    20

<210> 20
<211> 19
<212> DNA
<213> Artificial Sequence

<220>
<223> Description of Artificial Sequence:PCR primer

<400> 20
agctcctgga ttctgcggt                                                    19


<210> 21
<211> 19
<212> DNA
<213> Artificial Sequence

<220>
<223> Description of Artificial Sequence:PCR primer

<400> 21
gaaggagagg tggtgccct                                                    19


<210> 22
<211> 20
<212> DNA
<213> Artificial Sequence

<220>
<223> Description of Artificial Sequence:PCR primer

<400> 22
cctgcatcca ttgacagcca                                                   20


<210> 23
<211> 18
<212> DNA
<213> Artificial Sequence

<220>
<223> Description of Artificial Sequence:PCR primer

<400> 23
gcagtgggtg caaggagt                                                     18


<210> 24
<211> 19
<212> DNA
<213> Artificial Sequence

<220>
<223> Description of Artificial Sequence:PCR primer

<400> 24
atggccagct tgtgccgga                                                    19


<210> 25
<211> 18
<212> DNA
<213> Artificial Sequence

<220>
<223> Description of Artificial Sequence:PCR primer

<400> 25

ggcggaatgc atccagag 18

<210> 26
<211> 19
<212> DNA
<213> Artificial Sequence

<220>
<223> Description of Artificial Sequence:PCR primer

<400> 26
ctgtcagcac cctgacagc 19

<210> 27
<211> 23469
<212> DNA
<213> Homo sapiens

<400> 27
aagtcaaggc tgcagtaagc catgatcgcg ccacagcact ccagcctggg tgacaaagtg 60
agaacatagc tcaaaaaata aaaaaataaa tttgcacctg aggcaagtgt cacactcatc 120
ccaactcagt ctcaccttgc atgttttctg ccttgctggc tgtatgaccc tggacaagtt 180
acttaaccgc tcagagcctc gatttcctcc tctataaaat ggggataatc tctgtaactg 240
ctcacagttg gtagcagaat gcaatgaagt gaattcgccc tcacagagcc agcactgttc 300
ttggcacatg gtaatcttaa catatttttt cctacaggga ggcctggtgt caggccggga 360
gtggggtgga agggtcccaa aatggatgga agggccccaa aatggccgtg agcatcctct 420
gcccttgaga agagctagcc cagctgtcta gagctccctg ctgctgccgc tctcgtaagc 480
agcaagcatt tttggctctc ctgtctcagc atgatgcccc tacaaggttc tttcgggggt 540
gggacccaac gctgctctcc tgatggcctc cctggctccc agcaccttcc atcccagctg 600
ctcagggccc ctcacctgcg cctcccccac cctcccctct gcccactccc atcgcaggcc 660
atagctccct gtccctctcc gctgccatga ggcctgcact ttgcagggct gaagtccaaa 720
gttcagtccc ttcgctaagc acacggataa atatgaacct tggagaattt ccccagctcc 780
aatgtaaaca gaacaggcag gggccctgat tcacgggccg ctggggccag ggttgggggt 840
tgggggtgcc cacagggctt ggctagtggg gttttggggg ggcagtgggt gcaaggagtt 900
tggtttgtgt ctgccggccg gcaggcaaac gcaacccacg cggtggggga ggcggctagc 960
gtggtggacc cgggccgcgt ggccctgtgg cagccgagcc atggtttcta aactgagcca 1020
gctgcagacg gagctcctgg cggccctgct cgagtcaggg ctgagcaaag aggcactgat 1080
ccaggcactg ggtgagccgg ggccctacct cctggctgga gaaggccccc tggacaaggg 1140
ggagtcctgc ggcggcggtc gaggggagct ggctgagctg cccaatgggc tgggggagac 1200
tcggggctcc gaggacgaga cggacgacga tggggaagac ttcacgccac ccatcctcaa 1260
agagctggag aacctcagcc ctgaggaggc ggcccaccag aaagccgtgg tggagaccct 1320
tctgcagtaa ggagccctgc cccgtccccg ctcccaggag agcctagagg ggcccccctc 1380
agctcctaac gagcccccct tctgagttga gtccccatga ccttcagcct ttagcctagt 1440
tgctgggaag gggacagggg cccatgagag cccaggggtc cttgcttgga ggtttgagcc 1500
tccagcccct gaactgctcc tctgcagagt cccaaatccc atgagcccag gcctttagcc 1560
cagtccttgg gcaaggggga catttcccag gggggtccaa gatgggagaa aaagcaggtg 1620
agttcacaac tcaaatgcct ggagaactgg gaggtggtgg aaatttttag ccactctgat 1680
catttatact ctccagaccc taactcctgc actgagtcct cagtgggtag gctccggggc 1740
ccaggaagca ctgccaggga ggagggcccg aagctggggt ttgggggctt tcctggcctc 1800
ctagggactc atgcgtttag ccagggaagc ccaggtcttt cttacctgag gcagacagag 1860
cctcgaggtg ggagctgctc ccctttcctg tatggccaca ggcaccccac ctcaccagca 1920
ggcgccatta gaggctgccc gttctacatc cccatccgct ggcggactcc ccgtctcctg 1980
gagaactggg tgggtcctga gctcgatgcc tcccctctcc tcagcagagc tgacctagtt 2040
actaattacc catgtgcttt attatttctc ttttgtttta taaatttata aatggcaaaa 2100
ggctctgata aggtctgtga tcattaactt gtaggaatgg tggcctggaa agatctgggc 2160
caacccacct cccacctagt gggggtgggg gatgggggtg tgggggagaa ggaaggatgg 2220
cctgggggct acaggggcca cccctcccca gcggctccct gacctccacc ctacagcgcc 2280
tgcttgagga ggttcactac ttcctgggcg ctgtgctgag gtttcacagg aataatttcc 2340
cttcatggtg aggagtcctg ggcagaagct attggtgact ctgcttcaca aatggggaaa 2400

ctggggctta gagccgggaa ggagctagac taagttcacc aaactgaggc cccaaaccta 2460
ctctgttcgg tgctctcaat tgctatgacc ggggaccaga ggcctgcagg gggtggccag 2520
ggagtcagag agagaccgag ccccaaagat gctccaaagg ggcccatctc ttttctgtcc 2580
aaggtcctga tcccagctgg ggtgggggag ggctggtgga ttcctgcttt cccagaggcc 2640
tcagggagcc tcccgagggc ctggacaggg ggcaggggag cagtcagggc cccctgactt 2700
ggcaggaaga ggagggcaaa ggactccaga gctgcagggg aggggcaaag agtcaggcat 2760
cccagaccca gggcgaaggc aacctgacgg ggtggggctg ggcctgacct gcccagggcc 2820
ccagatggga ctgggggctt tggggtgagg gtgggggcag gggatgcttt cttacctggc 2880
tgagtgagct ctctcggtca gcagccccct ttggtgggat ggtggggttg gggaggcttg 2940
atccacagca tttgaagggg aagcagaggt caaagtgctt cctagggacc agcagagacc 3000
tggaagctga ggcagagagt gtagagagag gccattgggg gagatggaca gagaagctgg 3060
gagaagcaga acggaggagc caggggggcg ggggctcaga cccagctggg agaaggggca 3120
cgggagagaa tgaggcgccc caagcttgca aggaggctgg agtgcttccc gcctgccccg 3180
attgggtttt ctttaatgct aacagcatgc tatttacttt ccatttaaat ttgagatgtt 3240
gctataaatt atcaaccagc tccttgttcc tgcagagttt ataactaact acctgggtta 3300
cttattgttc aggtaacaaa agggatcgga aaacgccctg agtgaaaaaa gtggggcctc 3360
cagcgtcagg gtcaggaaag gagccaggga gagaggggcg gggaccccct attgaaggcc 3420
tgggccattg gggaggttga ggctgggaag acggtacaga ggcagaatgt ctagtagaag 3480
ctgtttcccg ggagaagtca gtgtctggga agaagccggg gtgggtcctg ggtcccaggc 3540
ctccctgggg tgccccatgt tggtggccac agcaggaacc tgagctcttt cttcagatct 3600
cccccttagc tccctgagtg accttgggaa agtctgttcc tctccttctg cctcagtttc 3660
cctccctggc caaggaataa agtccaaact ccctgccttg gtttcgttcc cctccctcca 3720
ccccaccaaa ttcctggatc tcttcccagg tctttctctt cctctcggcc tttgctgcag 3780
tcattttggc tttgctgttc ctcagtaccc caagccaatt cccaccccag ggcctttgca 3840
cgtgctcttc ctgctgctca gaacctcttt ccagatcttc ccatggccga ttccttctcc 3900
ttcagctccc tgattgctcc atccaaaagg gggccccacc cccgtcactc tcttccacta 3960
ctgttgttca ctttctctgg ttccctgtca agacttgtga tcatctgatt tgtttgtttt 4020
ctatctcctg ccatgaaaat agaagctctt caaggacaag gcccgtgttg ccttatttac 4080
cacttgtacc cacatcctgc ccagtggctg atgcacagca ggaatgagtg agtgactggg 4140
attgtctgag gccctgaaga tatctgctgc cctgttggtc agggcccagc agcctgagac 4200
gtggccaagg gagaaactgg gacccagagt tccttcctgg gtgtcctccc gggtcctttc 4260
tgtccctgat cagctcaaac cccagcgtgt ttcttttttg ttgttgttgt tgttattttt 4320
gagacggagt ctcactctgt cggcccggct ggagtgcatg gcgcgatctc ggctcactgc 4380
aagctctgcc tcctgggttc acgccattct cctgcctcag cctcccgagt agctgggatt 4440
acaggcatgc accaccacgc ctggctgatt ttttgtattt tagtagagat ggggtttcac 4500
tatgttggcc aggctggtct caaactcctg acctcaggtg atccgcccgc ctcggcctcc 4560
caaagtgctg ggattacagg cactcagagc cactgccctc agccttcaaa ccccaatctt 4620
gaagtattct gatcctgccc atcaggcagc atcagggcac tgagactcag gaaaacagta 4680
gccacggcag gagggggggg ggacagagga tctgacgtcc ccaacaaaag ccagactgcc 4740
tgcatcctaa tcctagctct gcccctttct agcttgagca ggtgatttta tacttctgag 4800
cctcagtttc gtctgcaggg tagagatgac aatagccctt gtccacaggg ttaaatggca 4860
tggcacttgt agagtaaaca tgtagtaact gcaatataca gcagttatta ttagtgatcc 4920
cattttacag atgagcaaat taaggctgag agaggcagtg ccttaccgaa cgaaactctt 4980
ccaaatgact gaaggcaagc caggacttgg acctgggtcc tcctacctca cgctccatac 5040
agcaacctcg aggtcacact ttcccctccc cagtcattcc tgctgtttaa gttgaccatc 5100
aactcacttc catacagtgc tttggagttc acaaagggct tacaaaatgc gctaagctca 5160
ttttttcctc ctccccatct ctgaagtctg aaatatcatt agccccattt cacagatgcg 5220
gaaactgagg cacacggagg ttaagcaaag aatcaaggcc aggtctaaag cccccacact 5280
gagaagtcag ctcccctcca gatgtggaga tgaataccac aaccttgcat gccctgaaac 5340
cccaggggcc agagggcaag aggctcaaag ccttggacat ccgattgacc ctggaaaggg 5400
taggagagag aggaggaaga tgtgggcggg tcttcacttc tcctgggctc tgctgtgtgg 5460
ggccaagggt ctccagggct gcattgttaa gatgatgatg atgacaatat tattccacaa 5520
attcagcact tactgtgcac caggcattgc cctaagttca tcctccctat ttgaagctca 5580
tttaattttc ccagtagcag tgtgaggtag gggtggattt accccacgtt acagggcaga 5640
tgtgggtcat gcaagagcac gcgtggctag gtcacagtgg tgggatttga acccacattg 5700
gtttgactct caaacccatt agagtcaaag caggagatgc ctggagactt gtcctcttag 5760
ttctttcctt tttctttctt tctttttttt tttttgagat ggagtctcac tctgttgctg 5820
ggggtggagt gcagtggcgc agtcttggct cattgcaacc tccgcctccc gggttcaagt 5880
gatcctcctg cctcagcctc ctgagtagct gggaccacag gcatacgaca ccacacccag 5940
ctaatttttg tatttttagt agagacaggg tttcactgtg ttggccaggg tggtctctaa 6000
ctcctgacct catgtgatcc acccgcctcg gcctcccaaa gtgtggggat tacaggcatg 6060

agccaccacg cccagccttg gcctcttagt tctgagctag gacagttgga gaatctgccc 6120
tcaaaaatag tcaggccctt gaagactctg tgtgtgtgtg tgtgtgtgtg tgtgtgtgtg 6180
tttgtgtaag agacacacag agagacagaa gagagagagt gatagcaagg gagtgggggg 6240
tagggagaga gagacatcga ttcagtaggt tctagaaaaa agtggcacag gtggcttctc 6300
ccagaaaagg gaaagacatg ccctcttggt tctctgactc ctaactggca tttaaaattc 6360
acttttttgt tgtggatttt ttgcccaact ctactaggaa ttgtttaaaa gtggaacgtt 6420
cgtatcagct cactttaaca gtttgctttc cattttcaca ttcaaaagcc tgtcttcggt 6480
ttctaagggt tgcacatgta tttgtgcagg gtggctggcc tttgttttat tttcaagttc 6540
agcttaaatt tgacctggca tgagagaatg agaatgagag agagagagag agaaagaaac 6600
agacagacat ggaatcagat ggacaggggc acatagaaaa tctttggttt gagcccagga 6660
gaggctctca agaggtgaga ggaagactga ccctattctc tttgtgggag agagaaggga 6720
aggagggagg aagggaggac acaggaggac atggcacagg agggtgatgg ctgcagatgg 6780
gggaggggag gcatcaggca gcctggctct aggatcagat aaacatgttt gccacagagc 6840
accaggctgg actttgtact gcccttcctt ctgagctctg actggcactc agcaaagcag 6900
ggagatgaag gagagaggat cctaggagcc ccggaaagaa gggagagaga gaagctgggg 6960
agaggcctgc gggggtgggg tggtcctggc aacatgaaat atttttgagt cccaactaga 7020
tcaccttgca caagtgactg cacctttctg agcctcagtt ttctcctctg caaaatgggc 7080
acaccgtcta cttcctaggg ttataagatg taagtggcca gtcctcagca cggtgcctgg 7140
ctcacagtaa atcctcaaga aacatgagtt ctcagacttt tttttttttt tttttgagac 7200
aggatttcac tctgtcgcca aggctggagt gcagtggcgc gatcacagct cattgcagcc 7260
ccgacctctc gggctcaaga gatcctcttg tctcagcctt ctaagtagct gggactacag 7320
gcttgtgcca ccacacttag ctaatttttc tattttttgt agaaacaggg tcccactatg 7380
ttgtccaggc tggtctcaaa ctcctgggcc caaggaatcc tcccacctta gcctcctgag 7440
gagctgggac tacaggtgtg caccaccatg cctggctaat ttattttttt ttttatagag 7500
acagagtctc actatgttgt ccaggctggt cttgaactgc agggctcaag tgatcctccc 7560
acctcggcct ttcaaagagc tgggattacc accatgagcc accataccca gtttacaatt 7620
cagttttaaa tattatatat gccaaggtgg gaggatcgct tgagcccagg agtttgagac 7680
cagcctggcc aacatagcaa gaccctgtct ctatgaaaaa aaaaaaatag aaaaaagaag 7740
aaaaagtaca aaaattagct gggcatgttg gcatgtgcct gtggtcccaa gtacttggga 7800
gtccgaggtg ggagaaccaa ttgagcccag gagtttgagg ctacagtgag ctatgatcac 7860
gccactgtgc tccagcctgg gagacagagc aagtagcctc taaaaagaaa aataaataaa 7920
taaaaataaa tattggctgg gcacggtggc tcacacctgt aatcccagca ctttgggagg 7980
ccaaggcaga tggatcacct gaagtcggga gtttgagacc agcctggcca acatggtgaa 8040
accctgtctc tactaaaaat ataaaaatta gctgggtgtg gtagcgcaca cctgtaatcc 8100
cagctactcg ggaggctgag gcaggagaat cgcttgaacc tgggaatcag aggttgcagt 8160
gagctgagct cgcgccactg cactccagcc tggccacaga gcaaggcttt gtctcaaaaa 8220
aaaaaactaa ataaaaataa atatcatgta tgcatatata gtatatatta tgtaatttac 8280
aaagtacaca aggacaacag agaaaactgt ccttctcact cccatcccca gcaccccgtt 8340
ctcctctgga aggaaccaga gtccccagtt ccttgcgtgc cctccggtat tttacacgtg 8400
cacaaatatg tagaaatcct cttttacctc acttcaaatt attcataagt agcagcacag 8460
cagacacacc agataaactc tgctccacct gggcacacag agcttcctca gcggggtttt 8520
ttcagtggtt gcattgaaca gatgcacctc catttagtta atcagtatcc ttttgctggg 8580
ctccttggtg gcttccaatc ttttgctatg gcaagcaacg tggcaaggaa ttgccttcta 8640
cttgtgccat ttgtcacctg tgccaatatt aaaagccaca aattctagaa gcgaattgct 8700
cagcgaggca ggggaaagca caggctttgg aatcacgcag acccaagttc aaatcccagt 8760
ctgcctgttt cagtgctgct gtattgagct gtatttatct gtaacttttt gtttgtttgt 8820
ttgttttttg agacggagtc tcactctgtt gcccaggctg gaatgcagtg gcgcgatctc 8880
ggctcactgc aacctctgcc tcccgggttc aagcaatcct cctgcctcag cctcccgagt 8940
agccgggatt acaggcacct gccacaatat ctgacttatt ttttattttt attatttttt 9000
tcgtagagat ggttttctcc atgttggcca ggctggtctt gaactcctaa cctcaaaaga 9060
cccgcccacc ttggcctccc aaagtgctgg gattacaggt gtgagtcact gcacccagcc 9120
tatctgtgac atattggtaa tataagttca ggagagggag agagaaagag gcagggattg 9180
agacagagca ggagaggagg agagaaaatt tatatggctc aggcagtctg atcccttctg 9240
ttcccccaca gggagaccca cagcagagac atgactcaca ggtggcatca ggtccctttg 9300
agtctctctg gtgggagaat ctcaacccac agagtaggat tccagtgttc acatgcattt 9360
ttggtactat gaggcctctg aatgtcaacc ctgtcacctg agactctgtt gaaaaaccag 9420
ccgcggccgg gcgcggtggc tcacgccagt aatcccagca ctttgggagg ccgaggcagg 9480
cagatcacaa gatcaggaga tcgagaccat cctggctaac acggtgaaac cccgtctcta 9540
ctaaaaatac aaaaaaatta gccgggcgtg gtggcgggtg cctgtaggcc cagctacttg 9600
ggaggctgag gcaggagaac ggcatgaacc caggaggtgg aacttgcagt gagccgagat 9660
cgcgccactg cactcgagcc tgggtgacag agtgagactc catctcaaaa aaaaaaaaaa 9720

aaaagaaaaa gaaaagaaaa accagcctcc tgttctggtg ctgcagggaa acaggcctgg 9780
ccacagccaa gggtgcagat tttcaggaag catttttaaa aatatatata tatatatata 9840
cacatatata tatatacaga gagagagaag ccactgaggc ccacagaatt tgcatcattt 9900
tattccttgt ccaaggtcac aggacaagca gagtcccacc cacctgaaag gattcagttc 9960
taagacagcc tttagggcaa aaagtcacaa agttgatcta tctatctatc tatctatcta 10020
tctatctatc tatctatcta tctacgtgtt tgcaccctaa tcactggcag taaatgcact 10080
tttttctttt tcttttcttt tttttgagac agggtcttgt tctgtcaccc aggctggagt 10140
aggtgtaaaa ttggagaatc catatttctt tccttgtttt cagatttaat gctttccttt 10200
ttccagcagg tctccttcgt ccatccatcc atcatccatc tacccaccca cccaccaatc 10260
catccatcca ttcatccaac catccatcca tccatcatcc atccacccac ccacccacca 10320
atccatccat ccattcatcc aaccatccat ccatccatca tccatccacc cacccaccca 10380
ccaatccatc catccattca tccaaccatc catccatcca tcatccatct acccacccac 10440
ccaccaatcc atccatccat tcatccaacc atccatccat ccatcatcca tccacccacc 10500
cacccaccaa tccatccatc cattcatcca accatccatc catccatcat ccatccaccc 10560
atctatctgt ccatccactc taccctacca tccatccacc agtccatcca tctatccacc 10620
aattcatcca tccatccacc cattcgccca tccatccatc cacccaccca tccatccatc 10680
cgtccatcca cccattcatc cattcatcca ttcacccatc catccatcca catatcttca 10740
tctgtgttgt gtgtctgtgt atccatgttt ctaaaccttt atctgttcca gtgtctgtat 10800
ccataggcct gtgtccacgt ttgtcatgtg tgtgcgtcta caagtctctg tcctcatgac 10860
catgtgtctg tgtccctgtg tcctggcata aatgaccata cctcaccgtc cctgagtcta 10920
tgtgtaggcc cctgggctcc ataactgctt tcatgcacag tccccaccct cagggttgac 10980
aaggttccag cacccaggac cgcagcccca cctatgggga gagacagccc ttgctgagca 11040
gatcccgtcc ttgccctctc ccagggagga cccgtggcgt gtggcgaaga tggtcaagtc 11100
ctacctgcag cagcacaaca tcccacagcg ggaggtggtc gataccactg gcctcaacca 11160
gtcccacctg tcccaacacc tcaacaaggg cactcccatg aagacgcaga agcgggccgc 11220
cctgtacacc tggtacgtcc gcaagcagcg agaggtggcg cagcgtaagt aatgacccta 11280
ccccgcatct tccctgggag ggcccaggac tctcccctaa ctcataggtg ggggctggaa 11340
gcttcaccat ccccattaca cagacaggta gatggaaagg aagtcagtgg gattcaacct 11400
gcatttatta cctattctgc gccaggcact ctgtgggacg ggagtagact tggtcctgaa 11460
catccaaaga tgaatgaaat gggtccctgc tttctttttc tttttttaga tagagttttg 11520
ctcttattgc ccaggctgga gttcagtggt gcgacctcag ctcactgtga cctcctcctc 11580
ccaggttcaa gcaattctcc tgcctcagcc tccggaatag ctgggatcac aggtgcccac 11640
caccatgcct ggctaatttt ttgtagtttt agtagagacg ggggtttcac catgttggcc 11700
aggctggtct cgaactcctg acctcaggtg atccacccac ctcagcctcc caaagtgctg 11760
ggattacagg cgtgagccac catacccggc ctgggtccct gctttctcag agcccatggt 11820
taagtgggaa agtgggagaa aagctcatta tgatccagcg agtcatgtat tagaatggga 11880
caacagcagc cacaacaaca acaataatgg ccactattta tcatgcattt ttggggtgcc 11940
tggtccagtg cagagtatta cacaaagcat ttcagggtgc tgagctgact gtcgactggg 12000
gagtaacctg ttcttcctca ggaaggcaga ggtatcagga agcctcacat tgcaaatatg 12060
acctttagaa tatgaccttt agactaggtc ttaaagtggg agttgaattt tgacccatgg 12120
agacgcaggg aagggtgttt caggcagaag gaacagcatc agtaaaggct cagaggcaag 12180
gtctacggtc aggaaatcct ccactgcaaa ctcaggaagc tctgggagcc ctattactca 12240
gggaatggtg ggttttcttc tccaggcctc ataccccacc aaatcccaag ggcactgtca 12300
ccttccaacc tccatctatg tgtgacattt tttgacattt cttttattaa ttatccaggt 12360
taattttttt ttattatttt tttatttttt tgagacagag tctcactctg ttgcccaggc 12420
tggagtgcag tggcgtgatc tcggctcact gcaagctccg ccttccgggt tcatgccatt 12480
ctcctgcctc agcctcccga gtagctggga ctacaggtgc ccgccaccac gcccagctaa 12540
ttttttgtat ttttagtaga gtcggggttt caccatggtc tcgatctcct gacctcgtga 12600
tccgcccgcc ttggcctccc aaagcgctgg gattataggc gtgagccacc gcgcctggcc 12660
ttaattatcc aggtttttaa aaaaggaaat agccaggcat ggtggtgtgc cataatctca 12720
gctactgagg aggctgaggt gggaggactg tttgagcccc caggggttcg gctccaacct 12780
ggacaacatg gcaaggttcc tcctctaaaa aagaaagaaa ggaaagatga taggaaaggg 12840
aggaaagata ggaaagggag gaaagatagg aaagggagga aaggtaggaa agggaggaaa 12900
ggtaggaaag ggaggaagga aggaaggaag gaaaagaaaa gaaagaaaaa gaacgagaga 12960
aagaaagaaa ggcaatacac atttggttaa aaaaaaagaa gaaaaacaga gtttataaga 13020
aaaattagat gcctttaccc taaagcagcc actggaattc ccagactctt gtgtatcctt 13080
ccagagagat tttctgcata tagtagcaat agatatgtta ttttcttgct ctttttctga 13140
ttaatgggaa catactagac acactatttt acatgttgtg ttttgtctct tggcaataaa 13200
tcctgtatat cttttcatat tggcactgag agattgatgg atctcattct ttataagggc 13260
tgctggctgt tatttgttta actggccacc tactgaagga cttttaggga ttttgcaatc 13320
tgcgaataca accaactgca gcaaaaaaca tccttcttta tataagtgca tgttcacatg 13380

tgactgtgtc tgtaggataa attcctagaa gtggactgct ggaacacaga gtagctgctt 13440
taaaatcttg ggtacatatt ggcatagttc cttccaaaga agttatatca atgtacactt 13500
ccaccagcag taaaaatcac tgttgaaaaa aaaaatgtat gcaggccggg cgcagtggct 13560
catgcctgta atcccagcac tttgggaggc caaggcaggt ggctcacttg agggcaggag 13620
tctgagacca gcctggtcaa catggtgaaa ccccatctta actaaaaata caaaaattag 13680
ctggacatag tggcgcatgc ctgtaatccc agctacttgg gaggccaagg caagggaatc 13740
gcttgaaccc aggaggcaga ggttgcagtg agccaagatc acgccactgc actccagcct 13800
gggtgacaga gggagactct gtcaaataaa tgtatgtatg tatgtatgta tgtatgtatg 13860
atgtatgtat gtatgcatgc atgcatgcat gcaatagaca actctagtcc ttactctata 13920
gctacccctc atcccaatta ttggggtgtt cacactctac tgtgctgtaa tacacacttg 13980
gaaaaacagt acattttaat actattttaa tttgtatgag aagaaacaaa gatttattct 14040
gaaaatctgt tagaactaga tttagtttct gagagaaata gtgttctcct aaaaccatca 14100
ctagacgaaa ttttacaata ttcgataaaa tgcattaaaa aatttctaac agttagaaat 14160
gtcatcaagg acttcagcct agagctgaga atagagatgc aaactgtggc ccaaatagct 14220
ttccctgggg gatttttaac ttttaaggag aaattggaga atttgagatg cagagtcaat 14280
atatgactgt agtattagga ctctttcagt aacaggggac agaaccccaa ctcaaatgga 14340
tttaaacaga aaagggaatg aattgactga agcaatgtga ggagtccagg gctgcttcag 14400
gcatggctag atcaaggggc tcaaatgagg ttcacacagc atttcttgac tcttttcttt 14460
tttgttggct tcattctcag acaggctctc ctcatatagg caaagatggg ttctggaagc 14520
cctgggctgc catcctacca gcttagcaac cttggaagga aaagagctct tcttttcagc 14580
tactttcagt tgaatgactc tttttatact tgataatgta aacttttatt tattttattt 14640
tttgagatga ggtcttgcca tgttgcccag gctagttttg aacttccggg ctcaagtgat 14700
ccctgcctca gcctcttaag tagctggaat ggcaggcaca catcactgtg tctgctataa 14760
cagaaacttt taaatgaagt ctaacataaa aagtgcccaa atccaaagca tacagttcaa 14820
tgaattttca caaagtgaac acaccaatac agataaaaaa tagaatatta ccagctccca 14880
gcctggtgtg gttgtaccca cctgtaatcc cagcacattt gggaggcaaa agtgggagga 14940
tcacttgagc ctaagagttc aagaccagcc tgggcaacag aaggagaccc catctctcca 15000
aaaaatacga ataaatgagc caggtgtggt gcagcaggtg tgtagtccta gccacttatg 15060
cccgctgctg tttcttggca ctgagatggt gagggccctg ctgctgctgc tgcgcccctg 15120
attcagaatc taccttcctc tcttctcata aagtgctgtc ctagcacttg tgtgtcccag 15180
tcctttggtg gcctgctcag ggataagctg gtagaccagt ttccagtaaa gtggtaccag 15240
ctgatgaagg ctggctgggt ctcttcctct agctaacatt tgcattttaa gcctgatttt 15300
caagtctgga aagctgaata taattctgaa tacactgagg acatatggct caaatttttg 15360
ctctactgcg tgactctgga aaaatatgta agctctctga gcctcagctt cctcatctgt 15420
acaatgggga tagtaaatgt gccaaatcag aacaaatgct aatgcttacc tgcagtcttg 15480
tactgagaag gatggtgaga tcatatcttg ggttggtagg aaagcattca gggattgatt 15540
agtgatgttt gccttgaaca caggttaaga aagtgatggc atgtgtgctg tgtgtttgtc 15600
atcagtagat tagatgattt ctaagttcta gctgtaagct cctctggttc agcgccatgg 15660
caatgagaaa gaatcaaggg caaggtcagg ggaatggacg tgggaaggtg agagtggcca 15720
gtaccccact cacggctttc tgtgcctgca gagttcaccc atgcagggca gggagggctg 15780
attgaagagc ccacaggtga tgagctacca accaagaagg ggcggaggaa ccgtttcaag 15840
tggggcccag catcccagca gatcctgttc caggcctatg agaggcagaa gaaccctagc 15900
aaggaggagc gagagacgct agtggaggag tgcaataggt acaacggcgg gcgggaaaca 15960
gtgctggttt ggtctgggct gcggcaaggc cagggaaggg gaaggtgact ctaggtcctg 16020
taaaaggctg tccagttgcc gagaactcct gatattggct tagcctggcc cagaaaattg 16080
agaatacttg aacctaagcc cattcctcgc agcccccctg caccctggac accaagcaac 16140
cccttccatg gatgctcacc caattcgatt ctctctacaa tcctatggct cttttgctca 16200
ctttatgaat ggagagactg aggtcagaca gactgtcaat tgcccaaggt cacacagcag 16260
acctggcatt ggaacccaga tctgccagcc tcaaaccctc cggcagagct cagcttctca 16320
gaaccctccc cttcatgccc aggacagggt tcctctgagc ctggcctgga ggctcatggg 16380
tggctatttc tgcagggcgg aatgcatcca gagaggggtg tccccatcac aggcacaggg 16440
gctgggctcc aacctcgtca cggaggtgcg tgtctacaac tggtttgcca accggcgcaa 16500
agaagaagcc ttccggcaca agctggccat ggacacgtac agcgggcccc ccccagggcc 16560
aggcccggga cctgcgctgc ccgctcacag ctcccctggc ctgcctccac ctgccctctc 16620
cccagtaagg tccacggtaa agtggtatgt ggggacaagg gacacgtggg aaggtgggag 16680
ggttggggag gactgtccca gtgacagcag tcacctaaac ctctttgcac ttcagtttgg 16740
ttccattcca ttcatgccac tccttatcac tctacttcac tctgttcatt catccattcc 16800
actctatctc attccattca ctctactcct ttccactcta ttcactccat ccaccacaat 16860
taaccccatt ccatccactc catccactac cttcgactcc actccatcca ctctactcca 16920
ttcactccac tcaactccac tccatccact ccactccgtc caacttcatc ccatccacta 16980
cattcaactc cactccatcc actctactcc atctactacc ttcaactcta ccccatccat 17040

ccactccact ccatccattc catccaactt catcccatct actacattca actctactcc 17100
atccactcca ctccatccat tgcatccaac ttcatcccat ctactacatt caactccact 17160
ccatccactc cactccatcc attccctcca acttcatccc atccagtaca ttcaactcca 17220
ctccatccac tgtactccat ctactacatt caactctact ccatccactc ccctccatcc 17280
attccatcca acttcatccc atctactaca ttcaactcca ctccattcat tccactccat 17340
ccattccatc caacttcatc ccatccacta cattcaactc cactccatcc actccatacc 17400
tggctccatc cactccactc catctactac attcaactct actccatcca ctccatactc 17460
tattccatcc acttactcca tccactccat tcagctccac tccatccact ccactcactc 17520
aactccatct actccactcc ctctacttca ttcaactctg ctccatccac tccactccac 17580
ccattccatc cactgcactc caaccagctc cattagactc cactccatcc actctaccca 17640
ctcttctctc cactcctctc cactacatac cattttattc tatctgtccc atccactcaa 17700
ctccattcac tccacatgac tccacatttc atccattcca ctctacttca tccactcact 17760
ccactctata ccattccact ccactctatt cacatactcc accattccag tctactccat 17820
tcactccact ccaacccact cactccactc cataccattc cactccactg tgttcacaca 17880
actccatcca ttccactcta gccactccat tcattccact ccacgccaca ctattcctca 17940
ccattccatc cactccaccc tataccattc cactccactc tattcctccc cacccgtcct 18000
ctccaccctt taccactcca ctcgactgta cccattccac ttgatcccac tcattccact 18060
caattccatc tactctactc cacaccatcc actccacttc atatcattcc actcaactca 18120
acctaagttg atttgggtta attcaattca attcattcat ttcagattgt atcaattcaa 18180
ttcatttcaa ttcaactaaa ttcagttaaa ttcagttcag ttgtcttcta ctgagcacct 18240
actgcatgtc aggtatagca ctaggcagtg ggaggaatgg agctaataaa tgcagtccca 18300
gccttcaagg aactgggagc agctgaccca gggcttggca aaaggtagaa acaaaggcag 18360
atttgctggc tgcataaagg cagacaggca gctggcctaa gcaaaccaat ggagtttgaa 18420
gtgctgaggg ctgtggaggc aggggagggc agggaagtgg ggtgctgagg caggacactg 18480
cttccctctc caggtgtgcg ctatggacag cctgcgacca gtgagactgc agaagtaccc 18540
tcaagcagcg gcggtccctt agtgacagtg tctacacccc tccaccaagt gtcccccacg 18600
ggcctggagc ccagccacag cctgctgagt acagaagcca agctggtgag tgtccttgct 18660
tgtaaggaaa acccaacctc atctttcctt ggcagggaga ttctggagca gtccctaggg 18720
aggccctgtg gggaccccgg ccccccggac acagcttggc ttcccctcgt aggtctcagc 18780
agctgggggc cccctccccc ctgtcagcac cctgacagca ctgcacagct tggagcagac 18840
atccccaggc ctcaaccagc agccccagaa cctcatcatg gcctcacttc ctggggtcat 18900
gaccatcggg cctggtgagc ctgcctccct gggtcctacg ttcaccaaca caggtgcctc 18960
caccctggtc atcggtaagc tggtggggat gggtgggcac ctgggtggga ggctcatggg 19020
gcaaccgcag aatccaggag ctggaagagc cactgggact cattcattca ttcatacaac 19080
atgtatttat ccagtgccta ctctggacca gtcactgtgc tacatcagtg atacctgggt 19140
gaaccaaaca gaccaaaatc tcagcaactc aagcagggag gcaggcacta agcataatac 19200
atcaattctg tggtacctca gaaggtgaag ggtctatggg caaattacag cagggtaagg 19260
gggactgatg ttttctaagt ttttgtttta tgaagaaaaa ttaagcccag aaagccctta 19320
tttgcaggta caattatgca gaagcccagt acataagata gataaggagc tgctaggaga 19380
ggggagcaga gaactgaccc catggccttt gcactgctgt ggaaccccag ggctccaggg 19440
aaccgcagtt tgacaacttt tgaacaagtc accgcctgcc tctcccacta gcctagacaa 19500
agagctaaag gctcagagag ggggaatgac ttgccagagc cacttaaatt agtggcaggt 19560
cccagtggag ggctgtttcc tgaccaccct gccccctcct ccaaaccacg ggctctggga 19620
aggagaggtg gtgcccttgg gaggtcttgg gcaggggtgg gatataactg gggggcccag 19680
ctgattccct ccccttccac tccaggcctg gcctccacgc aggcacagag tgtgccggtc 19740
atcaacagca tgggcagcag cctgaccacc ctgcagcccg tccagttctc ccagccgctg 19800
cacccctcct accagcagcc gctcatgcca cctgtgcaga gccatgtgac ccagagcccc 19860
ttcatggcca ccatggctca gctgcagagc ccccacggtg agcgccctgt gccccacaca 19920
gcaggagatg atgatagagg ttggctgtca atggatgcag gggaaagggg tgcctggcag 19980
gcattgcagt ctgcatgtgt ctctgggaca agtgtgtttc cgtgattgag ggtgtctgca 20040
ggccagtgtg ttcccatgtg aatgcacgta tctgtgtgtg tgcacgactg cttgtgtgag 20100
cagatcccta gtgcgtgtct gggtgtgtat cggttgtgca tgcatttgtg tgcatgcctg 20160
tgtttctctg aaactcttag ggccatatga atttctaaaa tctattcaga ttttagaaag 20220
gtaatctggg gccaggcgtg gtggctcatg cctgtaatcc cagcactttg gaaggccgag 20280
gtgggcagat cacttgaggt caggagttca agaccagcct ggccaacacg gtgaaacccc 20340
gtctctacta aaagtacaaa aattagccag gcgtggaggc acgtgcctgt agtcccagct 20400
acttgggagg ctgaggcaga atcgcttgaa cctgggaggc ggaggttgca gtgagctgag 20460
attgtgccac tgcactgcac tccagcctgg gcaacagagt gagtactctg ccaaaaaaaa 20520
aaaaaagaaa aagaaaagta atttggtgga tatgccagct atcacataac actcccagca 20580
gggtttgaga cagccccctg tgatcaaagc actgctattt ctgcagccaa agataggatt 20640
tccacaaggc agtttaaaca ctcataacct cataccaggt tcaggctggg tttcgccacc 20700

aaatgagctc tagtgccaat cttaaggaaa tcttttggtt tcagaccttt ttggaatttg 20760
ggactgggtt aagagatttg gggcctgtgt atgtctgtgt gtacatggtt gagagtgtat 20820
gcgtgtgtgt gtgttacatt tttatctgag tcactgtgag aatatgtggt ttatgtgcgt 20880
tttcctagtg tctgtgtgtc tcttgtaggg tctatgtgtc tcctgagggg tgggtggagg 20940
tgctgtaggc aaaacatggg gttatatttg gaaaggtggc ctggagatgg aagtactgct 21000
gtcctctgcc ttgctctttg acaggggcca cctgaactta ctacacagca cctgagttta 21060
gtacagtagc acctgtgctt agtacatagc acctgtgttt agtatagtag cacctgtgct 21120
tagtacatag cgcctgcatt tagtacagta gcacctgcgc ttagtacgta gcacccgtgc 21180
ttagtacagt agccctgcac ctgcacctcc cgttcccttt catacctgct gtctccaggt 21240
gacaagagga gctggagttg gtttctggcc tcctccaggc tcccctgcat caagcgcagc 21300
tgagcagttc cctgtaatgg ggagagggtc tgtcccttta tctggagcct ccagttttga 21360
aaatcagccc tggatctcca actgctgccc agtctggctg ttcagcaggc cccatgcccc 21420
cctttcccca gtcttgaggc ctgggactag ggctgtcagg cacgtctgcc acgtctgccc 21480
ctctctcccc tgcggccagc cctctacagc cacaagcccg aggtggccca gtacacccac 21540
acgggcctgc tcccgcagac tatgctcatc accgacacca ccaacctgag cgccctggcc 21600
agcctcacgc ccaccaagca ggtaaggtcc aggcctgctg gccctccctc ggcctgtgac 21660
agagcccctc acccccacat cccccgggct caggaggctg ctctgctccc ccaggtcttc 21720
acctcagaca ctgaggcctc cagtgagtcc gggcttcaca cgccggcatc tcaggccacc 21780
accctccacg tccccagcca ggaccctgcc agcatccagc acctgcagcc ggcccaccgg 21840
ctcagcgcca gccccacagg tgagaggccc tggctccacc ccctccctta ctgtccctgc 21900
ccccttccat gttggtccca ccccttctgt tgctgtccgt cactgtgggg ctgtgcatgc 21960
agcaggccta gggctgctgt gaggaagcac tggcaggcgt ggaggggtgg ggtgggcttc 22020
catgaagccc aagaggcaca ggcgacccca ggaagatggg gcccacctta caagaacatc 22080
tcagggactg gactgaggaa aatgatgaag taattgttag aggcagtgga gagtaacgat 22140
aaggaccagg tctgtgagcc agaagcgact cttgcatgtg tttggtttgg cttggctcag 22200
ggttgaacat tctttgaatt tgttgccaac atttcaaaat caggaatttt cacatagaaa 22260
tccagatttc tggcatcttt tggaaaattg gaaaaattca tattcccata taacagtgca 22320
ccaggcctca ctgcatccac tcacatcggc tccaatgacc cctggcggtg gggcggtcac 22380
ctgagtttgt aatctatgat gccattgtca ggagtgggca ctttggagtc aggtgggtgg 22440
gctttgcagc ctgccttttt gcaagctgcc ccctttctat aaggttcctg gacccggccc 22500
tggcagctgt catgacagcc actgaggcca ttctgtgtcc tggcattcac cccatgaggc 22560
gtgtccatga ccacccccat tttacagatg agaaagtgga cactcaagga ggggagggac 22620
ttgcctcatc tcccacagct accagatggc agggccagga ctcgaaccca ggtcccacct 22680
caaagcccat tctcttaacc attgccatca gtcagggttc tccaggaaca cagaaccaac 22740
agatttattt gaaggacttg gcccgtgtct gcggggggct ggcaggcctg aaatctgttg 22800
ctggcagctc tggggagtga attctgcagc cttgaggcag aattccttct ctgggaaacc 22860
ggtttttgct cttacggcct tccactgatg taggaggccc acccatattt tcagggtaat 22920
ctccttcact caaagccaac tgattgtggc attaaccaca tctacaaagc acctgcattc 22980
acagcagcgc ctagattagt gtttgactca gcctagccaa gccaacacgt acaactacct 23040
acctcggcat ctcaccgggg cttctccagt gttcacacta agatgtactc aggccactcc 23100
atgggcggcc gtggaccctg gctgggaggc tccctttgaa gaaccgaggg tagaggtgtg 23160
actttggggt tcctgttatc tgctgtgatc caggaggtgt ggccctgcct ccccatcctg 23220
agtaccccta gggacaggca ggtggggtgg gtgtgggtgc ctggtgggtg gctagcagcc 23280
ttgtttgcct ctgcagtgtc ctccagcagc ctggtgctgt accagagctc agactccagc 23340
aatggccaga gccacctgct gccatccaac cacagcgtca tcgagacctt catctccacc 23400
cagatggcct cttcctccca gtaaccacgg cacctgggcc ctggggcctg tactgcctgc 23460
ttggggggt 23469

## Description

The present invention relates to diagnostic methods and therapeutics in the field of tumors involving Hepatocyte Nuclear Factor 1α (HNF1α).

The Hepatic Nuclear Factor 1α (HNF1α) gene, also known as the transcription factor 1 or TCF1 gene, maps to chromosome 12. It is expressed in different tissues including liver, kidney, pancreas and digestive tract. More particularly this factor represents a crucial transcriptional activator of many hepatic genes, including albumin, β fibrinogen and α1 antitrypsin (Frain M. et al. (1989), Cereghini S. et al (1990), Baumhueter S. et al (1990), Chouard T. et al, (1990)). HNF1α is encoded by 10 exons, which span 23 kb. The protein is comprised of three functional domains. Exon 1 of the HNF1α gene encodes the domain necessary for dimerization. Exons 2, 3 and 4 encode the domain required for binding to DNA. Exon 5-10 encode the domain required for transactivation of transcription (C.R. Acad. Sci. Paris, Life Science (1993) 316:385-94). HNF1α regulates transcription of a particular gene by dimerizing and binding upstream of the gene's promoter. The transcription factor can form homodimers with itself or can heterodimerize with the protein product of a different gene known as vHNF 1.

In 1996, Yamagata et al. demonstrated that heterozygous germline mutations of HNF1α were linked to the occurrence of MODY3 (Maturity Onset Diabetes of the Young type 3, OMIM #600496) in humans. MODY3 is a dominantly inherited, rare subtype of non-insulin-dependent diabetes mellitus (NIDDM) characterized by early onset, usually before the age of 25, and a primary defect in insulin secretion. U.S. patents No. 6,187,533 and U.S. No. 6,143,491 propose that mutations in the HNF1α gene are associated with diabetes and as a result contemplate methods for diagnosing diabetes and methods for treating diabetes.

In a study of hepatic adenomas and hepatocellular carcinomas (HCC), the inventors have discovered that HNF1α was a tumor suppressor gene.

Liver adenomas are benign tumors at risk of malignant transformation. A genome wide search for loss of heterozygosity (LOH) revealed a chromosome 12q deletion in 5 of 10 adenomas. In most cases, 12q LOH was the only recurrent genetic alteration observed, suggesting the presence of a tumor suppressor gene therein. A minimal common region of deletion was defined in 12q24 including the HNF1α gene. Bi-allelic inactivation of HNF1α was found in 10 out of 16 screened adenomas and heterozygous germline mutation was present in 3 patients. Furthermore, somatic bi-allelic mutations were also found in two well-differentiated hepatocellular carcinomas (HCC), occurring in normal liver, out of 30 screened HCCs. These results reveal that HNF1α inactivation, whether sporadic or associated with MODY3, is an important genetic event in the occurrence of human liver adenomas and may be an early step in HCCs.

The inventors further studied the impact of HNF1α in colorectal carcinogenesis. Sequencing of all HNF1α exons with their intron-boundaries was performed in 10 colon adenomas and 29 colorectal carcinomas. Additional mutations were identified.

These results open new diagnostic and therapeutic pathways in the field of tumors and cancers.

Moreover, as liver adenomas are usually observed in young women using oral contraceptives, the inventors make a link between HNF1α alteration, cellular proliferation and the use of estroprogestatives.

### General definitions

The present invention is aimed at providing diagnostic and therapeutic methods in the field of tumors. In the context of the present invention, the term *"tumor"* means any abnormal proliferation of cells. According to the invention, the tumors are generally solid tumors, that can be benign or malign. Cancers are particularly encompassed. All types of tissues may be affected, including liver, kidney, pancreas, digestive tract (*e.g.* stomach, intestines, and especially colon), or gall-blader. Preferably the tumor is a liver tumor. More preferably the tumor is a hepatic adenoma or a hepatocellular carcinoma.

Hepatic or hepatocellular adenomas are solitary or less frequently multiple primary tumors usually observed in young women using oral contraceptives (Edmondson H.A. et al (1976)). Occasionally, their development is associated with the use of anabolic corticosteroids or with type I glycogen storage disease (Sale G.E. et al (1977), Howell R.R. et al. (1976)). Histologically, adenomas are well-circumscribed tumors with loss of the normal lobular architecture, composed of cells resembling normal hepatocytes arranged in cords or plates of one to two cells thickness, often with areas of dilated sinusoids. Multiple adenomas, or adenomatosis, is a separate entity where the adenomas have equal frequency in both sexes and are not influenced by oral contraceptive use. No significant histological differences are observed between solitary adenomas and those developed in hepatic adenomatosis (Flejou J.F. et al. (1985), Chiche L. et al. (2000)). A relationship between adenoma and HCC has been suggested by several clinical reports of malignant transformation of hepatic adenomas (Goldfard S. (1976), Ferrell L.D. (1993)).

Colon or colorectal tumors are also encompassed in the present invention. Liver and colon carcinogenesis are multi-step processes implicating common pathways such as activation of the Wnt signalisation, or the P53 and CDKN2A inactivation. In both tumor types, chromosome instability has been described (Lengauer, et al., 1997 ; Laurent-Puig, et al., 2001). Genetic instability defining the microsatellite instability phenotype (MSI-H) has been shown in colorectal cancer and accounts for 15% of colorectal cancer cases. The MSI-H phenotype is associated with mismatch repair deficiency leading to the accumulation of genetic alterations in targeted genes with simple nucleotide repeats in their coding sequence.

The *"subject"* or *"patient"* to be diagnosed or treated may be any human or non-human animal, preferably a mammal, including rodents, monkeys, cattle, sheep, feline, canine, horses, rabbits, goats,... Naturally the subject is of a species that expresses HNF1α and preferably is likely to be at risk to develop a tumor.

According to the present invention, *"HNF1α protein" "HNF1αgene" or "TCF1 gene "* refers to the human form or homologous (namely sequence-conservative and function-conservative) variants in other species. The cDNA sequence that encodes human HNF1α is shown in the sequence listing as SEQ ID No. 1. The corresponding amino acid sequence is shown as SEQ ID No. 2. HNF1α protein is a 631 amino acids protein composed of three functional domains: a dimerization domain (AA 1-33), a DNA-binding domain that includes a region (AA 100-184) necessary for specific DNA recognition and a homeodomain (AA 198-281), and a transactivation domain (AA 282-631) (Chouard et al, 1990 ; Nicosia et al, 1990; Schott et al, 1997 ; Vaxillaire et al, 1999). The full gene has been sequenced EMBL Access = HSHNF1; M57732; J04771 ; Swiss prot: P20823 ; RefSeq (NCBI): NM_000545).

The genomic sequence of the human HFN1α gene is shown in SEQ ID No 27.

HNF1α has been characterized in various other species too, including mouse, rat and xenopus.

A *"functional HNF1*α *protein"* refers to a protein that shows essentially the same activity or level of activity as the wild-type HNF1α protein. This activity includes the transcriptional activation of genes such as albumin, β fibrinogen and α1-antitripsin, by dimerizing and binding upstream of the gene's promoter.

A *«biological sample »* refers to any source of biological material (DNA, mRNA, or protein) available in practice. It includes without limitation body fluids, such as blood, urine, plasma, serum, cerebrospinal fluid, saliva, and biopsy tissues or surgical specimen. Liver biopsies are particularly preferred.

A *"promoter"* or *"promoter sequence"* is a DNA regulatory region capable of binding RNA polymerase in a cell and initiating transcription of a downstream (3' direction) coding sequence. The promoter sequence is generally bounded at its 3' terminus by the transcription initiation site and extends upstream (5' direction) to include the minimum number of bases or elements necessary to initiate transcription at levels detectable above background. Within the promoter sequence are found a transcription initiation site (conveniently defined for example, by mapping with nuclease SI), as well as protein binding domains (consensus sequences) responsible for the binding of RNA polymerase. The promoter may be operatively associated with other expression control sequences, including enhancer and repressor sequences.

Promoters which may be used to control gene expression include, but are not limited to, cytomegalovirus (CMV) promoter (U.S. Patents No. 5,385,839 and No. 5,168,062), the SV40 early promoter region the promoter contained in the 3' long terminal repeat of Rous sarcoma virus the herpes thymidine kinase promoter, the regulatory sequences of the metallothionein gene; prokaryotic expression vectors such as the beta-lactamase promoter or the *tac* promoter; promoter elements from yeast or other fungi such as the GaI 4 promoter, the ADC (alcohol dehydrogenase) promoter, PGK (phosphoglycerol kinase) promoter, alkaline phosphatase promoter; and transcriptional control regions that exhibit hematopoietic tissue specificity, in particular: beta-globin gene control region which is active in myeloid cells, hematopoietic stem cell differentiation factor promoters, erythropoietin receptor promoter, etc.

The terms *"mutant"* and *"mutation"* mean any detectable change in genetic material, *e.g.* DNA, or any process, mechanism, or result of such a change. This includes gene mutations, in which the structure *(e.g.* DNA sequence) of a gene is altered, any gene or DNA arising from any mutation process, and any expression product *(e.g.* protein) expressed by a modified gene or DNA sequence.

*"Sequence-conservative variants"* of a polynucleotide sequence are those in which a change of one or more nucleotides in a given codon position results in no alteration in the amino acid encoded at that position.

*"Function-conservative variants"* are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Such changes are expected to have little or no effect on the apparent molecular weight or isoelectric point of the protein or polypeptide. Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide which has at least 60 % amino acid identity as determined by BLAST or FASTA algorithms, preferably at least 75 %, most preferably at least 85%, and even more preferably at least 90 %, and which has the same or substantially similar properties or functions as the native or parent protein to which it is compared.

As used herein, the term *"homologous"* in all its grammatical forms and spelling variations refers to the relationship between proteins that possess a "common evolutionary origin," including proteins from superfamilies *(e.g.,* the immunoglobulin superfamily) and homologous proteins from different species *(e.g.,* myosin light chain, etc.) (Reeck *et al.,* (1987)). Such proteins (and their encoding genes) have sequence homology, as reflected by their sequence similarity, whether in terms of percent similarity or the presence of specific residues or motifs at conserved positions.

Accordingly, the term *"sequence similarity"* in all its grammatical forms refers to the degree of identity or correspondence between nucleic acid or amino acid sequences of proteins that may or may not share a common evolutionary origin *(see* Reeck *et al., supra).* However, in common usage and in the instant application, the term "homologous," when modified with an adverb such as "highly," may refer to sequence similarity and may or may not relate to a common evolutionary origin.

In a specific embodiment, two DNA sequences are "substantially homologous" or "substantially similar" when at least about 80 %, and most preferably at least about 90 or 95 %, of the nucleotides match over the defined length of the DNA sequences, as determined by sequence comparison algorithms, such as BLAST, FASTA, DNA Strider, etc. An example of such a sequence is an allelic or species variant of the specific genes of the invention. Sequences that are substantially homologous can be identified by comparing the sequences using standard software available in sequence data banks, or in a Southern hybridization experiment under, for example, stringent conditions as defined for that particular system.

Similarly, in a particular embodiment, two amino acid sequences are *"substantially homologous"* or *"substantially similar"* when greater than 80 % of the amino acids are identical, or greater than about 90 % are similar (functionally identical). Preferably, the similar or homologous sequences are identified by alignment using, for example, the GCG (Genetics Computer Group, Program Manual for the GCG Package, *Version* 7, Madison, Wisconsin) pileup program, or any of the programs described above (BLAST, FASTA, etc.).

A nucleic acid molecule is *"hybridizable"* to another nucleic acid molecule, such as a cDNA, genomic DNA, or RNA, when a single stranded form of the nucleic acid molecule can anneal to the other nucleic acid molecule under the appropriate conditions of temperature and solution ionic strength *(see* Sambrook *et al., 1989).* The conditions of temperature and ionic strength determine the "stringency" of the hybridization. For preliminary screening for homologous nucleic acids, low stringency hybridization conditions, corresponding to a Tm (melting temperature) of 55°C, can be used, *e.g.,* 5x SSC, 0.1 % SDS, 0.25 % milk, and no formamide ; or 30 % formamide, 5x SSC, 0.5 % SDS). Moderate stringency hybridization conditions correspond to a higher Tm, *e.g.,* 40 % formamide, with 5x or 6x SCC. High stringency hybridization conditions correspond to the highest Tm, *e.g.,* 50 % formamide, 5x or 6x SCC. SCC is a 0.15 M NaCl, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived *(see* Sambrook *et al., 1989,* 9.50-9.51). For hybridization with shorter nucleic acids, *i.e.,* oligonucleotides, the position of mismatches becomes more important, and the length of the oligonucleotide determines its specificity *(see* Sambrook *et al., 1989,* 11.7-11.8). A minimum length for a hybridizable nucleic acid is at least about 10 nucleotides ; preferably at least about 15 nucleotides ; and more preferably the length is at least about 20 nucleotides.

In a specific embodiment, the term *"standard hybridization conditions"* refers to a Tm of 55°C, and utilizes conditions as set forth above. In a preferred embodiment, the Tm is 60°C. In a more preferred embodiment, the Tm is 65°C. In a specific embodiment, "high stringency" refers to hybridization and/or washing conditions at 68°C in 0.2 X SSC, at 42°C in 50 % formamide, 4 X SSC, or under conditions that afford levels of hybridization equivalent to those observed under either of these two conditions.

The terms *"vector", "cloning vector"* and *"expression vector"* mean the vehicle by which a DNA or RNA sequence *(e.g.* a foreign gene) can be introduced into a host cell, so as to transform the host and promote expression (e.g. transcription and translation) of the introduced sequence. Vectors include plasmids, phages, viroses, etc.; they are discussed in greater detail below.

Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites. A "cassette" refers to a DNA coding sequence or segment of DNA that codes for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "DNA construct." A common type of vector is a "plasmid", which generally is a self-contained molecule of double-stranded DNA, usually of bacterial origin, that can readily accept additional (foreign) DNA and which can readily introduced into a suitable host cell. A plasmid vector often contains coding DNA and promoter DNA and has one or more restriction sites suitable for inserting foreign DNA. Coding DNA is a DNA sequence that encodes a particular amino acid sequence for a particular protein or enzyme. Promoter DNA is a DNA sequence which initiates, regulates, or otherwise mediates or controls the expression of the coding DNA. Promoter DNA and coding DNA may be from the same gene or from different genes, and may be from the same or different organisms. A large number of vectors, including plasmid and fungal vectors, have been described for replication and/or expression in a variety of eukaryotic and prokaryotic hosts. Non-limiting examples include pKK plasmids (Clonetech), pUC plasmids, pET plasmids (Novagen, Inc., Madison, WI), pRSET or pREP plasmids (Invitrogen, San Diego, CA), or pMAL plasmids (New England Biolabs, Beverly, MA), and many appropriate host cells, using methods disclosed or cited herein or otherwise known to those skilled in the relevant art. Recombinant cloning vectors will often include one or more replication systems for cloning or expression, one or more markers for selection in the host, *e.g.* antibiotic resistance, and one or more expression cassettes.

The terms *"express"* and *"expression"* mean allowing or causing the information in a gene or DNA sequence to become manifest, for example producing a protein by activating the cellular functions involved in transcription and translation of a corresponding gene or DNA sequence. A DNA sequence is expressed in or by a cell to form an "expression product" such as a protein. The expression product itself, *e.g.* the resulting protein, may also be said to be "expressed" by the cell. An expression product can be characterized as intracellular, extracellular or secreted. The term *"intracellular"* means something that is inside a cell. The term *"extracellular"* means something that is outside a cell. A substance is "secreted" by a cell if it appears in significant measure outside the cell, from somewhere on or inside the cell.

The term *"transfection"* means the introduction of a foreign nucleic acid into a cell. The term *"transformation"* means the introduction of a "foreign" *(i.e.* extrinsic or extracellular) gene, DNA or RNA sequence to a host cell, so that the host cell will express the introduced gene or sequence to produce a desired substance, typically a protein or enzyme coded by the introduced gene or sequence. The introduced gene or sequence may also be called a "cloned" or "foreign" gene or sequence, may include regulatory or control sequences, such as start, stop, promoter, signal, secretion, or other sequences used by a cell's genetic machinery. The gene or sequence may include nonfunctional sequences or sequences with no known function. A host cell that receives and expresses introduced DNA or RNA has been "transformed" and is a "transformant" or a "clone." The DNA or RNA introduced to a host cell can come from any source, including cells of the same genus or species as the host cell, or cells of a different genus or species.

The term *"host cell"* means any cell of any organism that is selected, modified, transformed, grown, or used or manipulated in any way, for the production of a substance by the cell, for example the expression by the cell of a gene, a DNA or RNA sequence, a protein.

The term *"expression system"* means a host cell and compatible vector under suitable conditions, *e.g.* for the expression of a protein coded for by foreign DNA carried by the vector and introduced to the host cell. Common expression systems include *E*. *coli* host cells and plasmid vectors, insect host cells and *Baculovirus* vectors, and mammalian host cells and vectors. In a specific embodiment, the protein of interest is expressed in COS-1 or C₂C₁₂ cells. Other suitable cells include CHO cells, HeLa cells, 293T (human kidney cells), mouse primary myoblasts, and NIH 3T3 cells.

*"Pharmaceutically"* or *"pharmaceutically acceptable"* refer to molecular entities and compositions that do not produce an adverse, allergic or other untoward reaction when administered to an animal, or a human, as appropriate.

As used herein, *"pharmaceutically acceptable carrier"* includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

### Diagnostics

An aspect of the present invention provides methods for determining whether a subject has or is at risk for developing a tumor.

A subject of the invention is an *in vitro* method for the diagnosis of a tumor in a patient, which method comprises determining the expression or activity of Hepatocyte Nuclear Factor 1α (HNF1α) in a biological sample, wherein an abnormal expression or activity in comparison with a control sample is indicative of a tumor or of a susceptibility to develop a tumor.

The step of determining the expression or activity of HNF1α may be performed according to various embodiments.

### Genetic lesion:

In a first embodiment, the step of determining the expression or activity of HNF1α comprises or consists in detecting a mutation in the HNF1α gene or in the promoter thereof, wherein said mutation results in impaired expression or activity of the HNF1α protein. At least one mutation may be detected.

The mutation maybe bi or mono-allelic. The inventors have more particularly found out that bi-allelic mutations could lead to hepatic adenomas or hepatocellular carcinomas, whereas mono-allelic mutations could result in colon tumors. Dominant negative mutations in HNF1α have already been described, in connection with MODY3 (*e.g.* mutation P291fsinsC, Yamagata et al, 1998, and Vaxillaire et al, 1999). These include mutants that encode proteins presenting an intact dimerization domain but forming non-productive dimers with wild-type protein, thereby inhibiting its activity.

The mutations are preferably located in the coding sequence of the HNF1α gene, or in the intron-exon splicing regions. Mutations of particular interest are shown in Table 2.

Preferred mutations include :
685C>T;617G>T;710A>G;436delC;82C>T;817A>G;spIVS2+1G>A;493T>A[495_526del];; 17del19bpinsT;872insC;196G>T;872delC;670C>G;379A>T;872insC;872del13;730A>G;618 G>T;1340C>T;26del17bp;803T>G;779C>T;872insC;632A>C;495G>T; and 749A>C.

Mutations 872delC(291frX342) and 872insC(291frX3156) are of particular interest especially in connection with colon tumors.

Mutations are herein described by giving the nucleotide position, and the change (>: substitution ; del : deletion ; ins : insertion ; sp : splicing site; IVS: Intron variant sequence followed by intron number ; in [ ] : mutation at the RNA level in the human sequence (SEQ ID n° 1)).

Mutations may also occur in the promoter.

Generally speaking, the mutation or genetic lesion may be (a) a deletion of one or more nucleotides from a wildtype gene; (b) an addition of one or more nucleotides to a wildtype gene; (c) a substitution of one or more nucleotides of a wildtype gene; (d) a gross chromosomal rearrangement of a wildtype gene.

The mutation is preferably a transversion G>T at nucleotide 617 resulting in an amino acid substitution W206L.

The mutation results in impaired expression or activity of the HNF1α protein. This means that the mutation results either in full inactivation of HNF1α, or in a diminished expression or activity of the protein. For instance, some mutants may have a reduced protein stability, whereas others may be defective in the DNA binding or impaired in their intrinsic trans-activation potential.

The detection of the mutation or genetic lesion may be performed by a variety of techniques well-known by the skilled artisan.

Genomic DNA used for the diagnosis may be obtained from body cells, such as those present in blood, tissue biopsy, or surgical specimen. The DNA may be isolated and used directly for detection of a specific sequence or may be PCR amplified prior to analysis. To detect a specific nucleic acid sequence, hybridization using specific oligonucleotides, direct nucleotide sequencing, RFLP, restriction enzyme digest, RNase protection, chemical cleavage, or ligase-mediated detection may be used. Oligonucleotides specific to mutant sequences can be chemically synthesized and labelled radioactively with isotopes, or non-radioactively using biotin tags, and hybridize to individual samples immobilized on membranes or other solid-supports by dot-blot or transfer from gels after electrophoresis. The presence or absence of these mutant sequences is then visualized using methods such as autoradiography, fluorometry, or colorimetric reaction. Cloned genomic or cDNA segments may be used as probes to detect specific DNA segments. Amplification of the nucleic acid, *e.g.* by PCR (polymerase chain reaction) or LCR (ligase chain reaction), can be used to enhance the sensitivity of this method.

More particularly, detecting the genetic lesion can include: (i) providing probes or primers comprised of an oligonucleotide which hybridizes to a sense or antisense sequence of an HNF1α, gene or gene fragment (wildtype or mutant); (ii) contacting the probes or primers to an appropriate nucleic acid containing biological sample obtained from the subject; and (iii) detecting, by hybridization of the probes or primers to the nucleic acid, the presence or absence of the genetic lesion.

In a preferred embodiment, the diagnostic methods utilize a set of primers for amplifying *(e.g.* via PCR or LCR) at least one region of an HNF1α gene, and means for analyzing the amplification product for differences *(e.g.* mutations) from the normal, wildtype coding sequence.

In another preferred embodiment, the diagnostic methods utilize a probe to determine its ability to hybridize under appropriately stringent conditions to a complementary nucleic acid sequence in the biological sample, wherein an inability of a probe, which is comprised of a wildtype HNF1α sequence, to hybridize to the sample nucleic acid is indicative of the presence of a mutation in the sample nucleic acid; or the ability of a probe, which is comprised of a mutant HNF1α, sequence to hybridize to the sample nucleic acid is indicative of the presence of a mutation in the sample nucleic acid.

Other nucleotide sequence amplification techniques may be used, such as ligation-mediated PCR, anchored PCR and enzymatic amplification as will be understood by those skilled in the art.

Sequence alterations may also generate fortuitous restriction enzyme recognition sites which are revealed by the use of appropriate enzyme digestion followed by gel-blot hybridization. DNA fragments carrying the site (normal or mutant) are detected by their increase or reduction in size, or by the increase or decrease of corresponding restriction fragment numbers. Genomic DNA samples may also be amplified by PCR prior to treatment with the appropriate restriction enzyme and the fragments of different sizes are visualize, for example under UV light in the presence of ethidium bromide, after gel electrophoresis.

Genetic testing based on DNA sequence differences may be achieved by detection of alteration in electrophoretic mobility of DNA fragments in gels. Small sequence deletions and insertions can be visualized by high resolution gel electrophoresis of single stranded DNA, or as changes in the migration pattern of DNA heteroduplexes in non-denaturing gel electrophoresis. Alternatively, a single base substitution mutation may be detected based on differential PCR product length in PCR. The PCR products of the normal and mutant gene may be differentially detected in acrylamide gels.

Nuclease protection assays (S1 or ligase-mediated) also reveal sequence changes at specific locations.

Alternatively, to confirm or detect a mutation or polymorphism resulting in restriction mapping changes, ligated PCR, ASO, REF-SSCP chemical cleavage, endonuclease cleavage at mismatch sites, SSCP or dHPLC (Denaturating High Pressure Liquid Chromatography) may be used. Both REF-SSCP and SSCP are mobility shift assays which are based upon the change in conformation due to mutations. SSCP and dHPLC are preferred methods of pre-screening particularly useful to detect mutations in the HNF1α gene.

DNA fragments may also be visualized by methods in which the individual DNA samples are not immobilized on membranes. The probe and target sequences may be in solution or the probe sequence may be immobilized. Autoradiography, radioactive decay, spectrophotometry and fluorometry may also be used to identify specific individual genotypes. Mutations can also be detected by direct nucleotide sequencing.

Preferably, mutations in the HNF1α gene are detected by PCR amplification of the screened region followed by direct sequencing of the purified PCR fragment using one of the primer used during PCR amplification.

According to an embodiment of the invention, the portion of the DNA segment that is informative for a mutation can be amplified using PCR. For example, the DNA segment immediately surrounding the 617G>T,W206L mutation acquired from tumor *(e.g.* adenoma) samples from an individual can be screened using the primers 5'-tagctgtaagctcctctggtand (SEQ ID No 3) and 5'-tacaggacctagagtcacct (SEQ ID No 4). This region is then amplified by PCR, the products are separated by electrophoresis, and transferred to membrane. Normal and mutant PCR products may then be detected using, for example, hybridization of labeled oligonucleotide probes and autoradiography, RFLP analysis, or direct sequencing.

### RNA assays:

In another embodiment of the invention, the step of determining the expression or activity of HNF1α comprises or consists in detecting a mutation in the HNF1α messenger RNA (mRNA) transcript, wherein said mutation results in impaired expression or activity of the HNF1α protein. At least one mutation may be detected. In that respect determining the presence of a non-wild type splicing pattern of a mRNA transcript of the gene may also be of particular interest.

The methods described above to analyze DNA may be adapted to analyze RNA. RNA may be isolated from a biological sample, *e.g.* a biopsy tissue, using standard methods well known to those of ordinary skill in the art such as guanidium thiocyanate-phenol-chloroform extraction. The isolated RNA may then be subjected to coupled reverse transcription and amplification (RT-PCR), using specific oligonucleotide primers that are specific for a selected site. Conditions for primer annealing are chosen to ensure specific reverse transcription and amplification; thus the appearance for an amplification product is diagnostic of the presence of a particular genetic variation. Alternatively, RNA is reverse-transcribed and amplified, after which mutations in the cDNA are identified by any technique as described above, *e.g.* direct sequencing. In still another embodiment, cDNA can be cloned and/or directly sequenced to identify a mutation.

In yet another embodiment, the step of determining the expression or activity of HNF1α comprises or consists in assessing the level of mRNA that encodes the HNF1α protein. Quantification of the level of HNF1α transcription can be determined using standard methods well known to those of ordinary skill in the art such as quantitative RT/PCR or northern blot analysis. An alteration in the level of a messenger RNA transcript of a gene is indicative of a tumor or of a risk to develop a tumor.

### Protein assays:

In still another embodiment, the step of determining the expression or activity of HNF1α comprises or consists in assessing the level of HNF1α protein or the level of activity of this protein. A reduced level of wild-type protein or a reduced level of activity of the protein is indicative of a tumor or of a risk to develop a tumor.

Determining the level of HNF1α protein may be performed by a variety of techniques. Immunodiagnostic techniques are particularly suitable therefore, including immunoassays such as competition, direct reaction or sandwich type assays. Such assays include, but are not limited to, Western blots, immunohistochemistry, agglutination tests, enzyme-labelled and mediated immunoassays, such as ELISAs, biotin/avidin type assays, radioimmunassays, immunoelectrophoresis, immunoprecipitation, etc. The reactions generally include revealing labels such as fluorescent, chemiluminescent, radioactive, enzymatic labels or dye molecules, or other methods for detecting the formation of a complex between the antigen (the HNF1α protein likely to be present in the sample) and the antibody or antibodies reacted therewith.

The term *"antibody"* in its various grammatical forms is used herein to refer to immunoglobulin molecules and immunologically active portions of immunoglobulin molecules, i.e., molecules that contain an antibody combining site or paratope. Exemplary antibody molecules are intact immunoglobulin molecules, substantially intact immunoglobulin molecules and portions of an immunoglobulin molecule, including those portions known in the art as Fab, Fab', F(ab')₂ and F(v).

Whereas polyclonal antibodies, that are easy to obtain, may be used, monoclonal antibodies are preferred because they are more reproductible in the long run.

Procedures for raising polyclonal antibodies are well known. Typically, such antibodies can be raised by administering the BARD1 protein subcutaneously to New Zealand white rabbits which have first been bled to obtain pre-immune serum. The antigens can be injected at a total volume of 100 µl per site at six different sites. Each injected material will contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed in Harlow et. al. (1988).

A *"monoclonal antibody"* in its various grammatical forms refers to a population of antibody molecules that contain only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, *e.g.* a bispecific monoclonal antibody. Although historically a monoclonal antibody was produced by immortalization of a clonally pure immunoglobulin secreting cell line, a monoclonally pure population of antibody molecules can also be prepared by the methods of the present invention.

Laboratory methods for preparing monoclonal antibodies are well known in the art (see, for example, Harlow et al. (1988)). Monoclonal antibodies (Mabs) may be prepared by immunizing purified HNF1α protein isolated from any of a variety of mammalian species into a mammal, *e.g.* a mouse, rabbit, goat, human and the like mammal. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody.

While Mabs can be produced by hybridoma culture, the invention is not to be so limited. Also contemplated is the use of Mabs produced by an expressing nucleic acid cloned from a hybridoma of this invention. That is, the nucleic acid expressing the molecules secreted by a hybridoma of this invention can be transferred into another cell line to produce a transformant. The transformant is genotypically distinct from the original hybridoma but is also capable of producing antibody molecules of this invention, including immunologically active fragments of whole antibody molecules, corresponding to those secreted by the hybridoma. See, for example, U.S. Pat. No. 4,642,334 to Reading; PCT Publication No. WO 890099 to Robinson et al.; European Patent Publications No. 0239400 to Winter et al. and No. 0125023 to Cabilly et al. In addition, the literature provides methods for forming chimeric antibodies, humanized antibodies, single chain antibodies and the like variations on a basic immunoreactive antibody fragment. All of these are considered within the scope of the invention insofar as a class and specificity of antibody is disclosed and claimed, regardless of the precise variant structure that one skilled in the art may construct.

Antibodies specific for the HNF1α protein can be purchased from Pharmingen or Santa Cruz Biotechnology. A set of polyclonal and monoclonal antibodies raised against the rat protein has also been described in Chouard et al, 1997.

Alternatively, one may assess the activity of the HNF1α protein present in the biological sample. This may be effected by any method known by the skilled person. For example, one may assess the ability of HNF1α to trans-activate down-stream genes, such as albumin, α and β fibrinogen and α1- antitrypsin. Reporter systems may be particularly useful for that purpose. According to one of these reporter systems, it is provided a chimeric construct, wherein the promoter of such a down-stream gene is fused or operatively associated with a reporter gene which expresses a polypeptide capable of producing a detectable signal. When the HNF1α protein is functional, it activates the promoter of said down-stream gene and causes a measurable detectable signal. The reporter gene may encode a luciferase, chloramphenicol acetyltransferase, β glucuronidase, β galactosidase, neomycin phosphotransferase, or guanine xanthine phosphoribosyltransferase, etc.

In still another embodiment the step of determining the expression or activity of HNF1α comprises or consists in detecting a mutation in the amino acid sequence of the HNF1α protein. This may be performed by different techniques used in protein analyses such as: western blotting, mass spectrometry or protein sequencing to detect HNF1α protein with aberrant migration or sequence pattern.

### Specific embodiments:

In a specific embodiment, the diagnostic methods of the invention make it possible to diagnose liver tumors, especially hepatic adenoma or hepatocellular carcinoma.

More particularly, the invention provides an *in vitro* method for the diagnosis of a liver tumor in a patient, which method comprises detecting a mutation in Hepatocyte Nuclear Factor 1a (HNF1α) gene in a liver sample, which mutation results in bi-allelic inactivation of HNF1α that is indicative of liver tumor selected from a hepatic adenoma and a hepatocellular carcinoma, or is indicative of a susceptibility to develop said liver tumor.

In another particular embodiment, colon tumors e.g., colorectal adenomas or carcinomas, may be advantageously detected by the methods of the invention.

More particularly, the invention provides *in vitro* method for the diagnosis of a colon tumor in a patient, which method comprises detecting a mutation in HNF1α gene in a colon sample, which mutation results in mono-allelic inactivation of HNF1α that is indicative of a colon tumor selected from a colorectal adenoma or carcinoma of the MSI-H phenotype, or is indicative of a susceptibility to develop said colon tumor.

In another aspect of the invention, it is provided a method for determining the susceptibility of a subject to develop a colon tumor, which method comprises detecting a polymorphism consisting of a mutation in the poly C tract repeat in exon 4 of the HNF1α gene (nucleotides 865 to 872 in the coding sequence), wherein a C(9) repeat *(i.e.* a polymorphic 864 G>C transversion) is correlated with a higher risk of HNF1α mutation, and as a result with a higher risk to develop a colon tumor.

In a further aspect of the invention, it may be advantageous to test any diabetic patient, especially a Maturity-Onset Diabetes of the Young type 3 (MODY3) patient, for its susceptibility to develop a tumor, e.g. a liver or a colon tumor.

The diagnostic methods of the invention may also be useful for female patients who use estroprogestative, especially as oral contraception.

### Therapeutics

Another aspect of the invention provides therapeutic methods in the prevention or treatment of tumors.

These methods are based on the discovery that HNF1α is a tumor suppressor gene whose expression is impaired in tumors. The purpose of the therapeutic methods of the invention is to restore or enhance the expression or activity of HNF1α protein.

The invention contemplates the use of (i) a nucleic acid that encodes a functional HNF1α protein or (ii) a functional HNF1α protein, for the preparation of a medicament for the prevention or treatment of a tumor.

The invention also contemplates a method for preventing or treating a tumor in a patient, which method comprises administering a efficient amount of (i) a nucleic acid that encodes a functional HNF1α protein or (ii) a functional HNF1α protein, in association with pharmaceutically acceptable carrier, in a patient in need a such treatment.

The first embodiment (i) involves gene therapy techniques, as discussed below. The second embodiment (ii), is a classic protein therapy, as described in greater detail below.

### Gene therapy:

In accordance with the present invention, the HNF1α nucleic acid, that is preferably a DNA sequence, forms part of a vector. Such vector is a nucleic acid comprising a HNF1α coding sequence operatively associated with sequences that control expression of HNF1α in a cell transfected with the vector.

The use of such a vector indeed makes it possible to improve the administration of the nucleic acid into the cells to be treated, and also to increase its stability in the said cells, which makes it possible to obtain a durable therapeutic effect. Furthermore, it is possible to introduce several nucleic acid sequences into the same vector, which also increases the efficacy of the treatment.

The vector used may be of diverse origin, as long as it is capable of transforming animal cells, preferably human tumor cells. In a preferred embodiment of the invention, a viral vector is used which can be chosen from adenoviruses, retroviruses, adeno-associated viruses (AAV), herpes virus, cytomegalovirus (CMV), vaccinia virus and the like. Vectors derived from adenoviruses, retroviruses or AAVs, HIV-derived retroviral vectors, incorporating heterologous nucleic acid sequences have been described in the literature (EP 185 573, WO 91/18088).

The present invention therefore also relates to any recombinant virus comprising, inserted into its genome, a HNF1α nucleic acid sequence as defined before.

Advantageously, the recombinant virus according to the invention is a defective virus. The term "defective virus" designates a virus incapable of replicating in the target cell. Generally, the genome of the defective viruses used within the framework of the present invention is therefore devoid of at least the sequences necessary for the replication of the said virus in the infected cell. These regions can either be removed (completely or partially), or rendered non-functional, or substituted by other sequences and especially by the HNF1α nucleic acid of the invention. Preferably, the defective virus nevertheless conserves the sequences of its genome which are necessary for the encapsulation of the viral particles.

It is particularly advantageous to use the nucleic acid sequences of the invention in a form incorporated in an adenovirus, an AAV or a defective recombinant retrovirus.

As regards adenoviruses, various serotypes exist whose structure and properties vary somewhat, but which are not pathogenic for man, and especially non-immunosuppressed individuals. Moreover, these viruses do not integrate into the genome of the cells which they infect, and can incorporate large fragments of exogenous DNA. Among the various serotypes, the use of the type 2 or 5 adenoviruses (Ad2 or Ad5) is preferred within the framework of the present invention. In the case of the Ad5 adenoviruses, the sequences necessary for the replication are the E1A and E1B regions.

The defective recombinant viruses of the invention can be prepared by homologous recombination between a defective virus and a plasmid carrying, inter alia, the HNF1α encoding sequence. The homologous recombination is produced after co-transfection of the said viruses and plasmid into an appropriate cell line. The cell line used should preferably (i) be transformable by the said elements, and (ii), contain sequences capable of complementing the part of the genome of the defective virus, preferably in integrated form so as to avoid the risks of recombination. As example of a line which can be used for the preparation of defective recombinant adenoviruses, there may be mentioned the human embryonic kidney line 293 which contains especially, integrated into its genome, the left part of the genome of an Ad5 adenovirus (12%). As example of a line which can be used for the preparation of defective recombinant retroviruses, there may be mentioned the CRIP line. Alternative vectors, such as shuttle vectors, can also be used that permit the cloning of the desired gene in the vector backbone.

Then the viruses which have multiplied are recovered and purified according to conventional molecular biology techniques.

Targeted gene delivery is described in International Patent Publication WO 95/28494.

Alternatively, the vector can be introduced in vivo by lipofection. There has been increasing use of liposomes for encapsulation and transfection of nucleic acids in vitro. Information regarding liposome is provided in the "pharmaceutical composition" section of the present application as well. Synthetic cationic lipids designed to limit the difficulties and dangers encountered with liposome mediated transfection can be used to prepare liposomes for in vivo transfection of a gene encoding a marker. The use of cationic lipids may promote encapsulation of negatively charged nucleic acids, and also promote fusion with negatively charged cell membranes. The use of lipofection to introduce exogenous genes into the specific organs in vivo has certain practical advantages. Molecular targeting of liposomes to specific cells represents one area of benefit. It is clear that directing transfection to particular cell types would be particularly advantageous in a tissue with cellular heterogeneity, such as pancreas, liver, or kidney. Lipids may be chemically coupled to other molecules for the purpose of targeting. Targeted peptides, e.g. hormones or neurotransmitters, and proteins such as antibodies, or non-peptide molecules could be coupled to liposomes chemically.

It is also possible to introduce the vector in vivo as a naked DNA plasmid. Naked DNA vectors for gene therapy can be introduced into the desired host cells by methods known in the art, e.g., transfection, electroporation, microinjection, transduction, cell fusion, DEAE dextran, calcium phosphate precipitation, use of a gene gun, or use of a DNA vector transporter.

### Protein therapy:

In another embodiment, the present invention provides a method for preventing or treating a tumor, which method comprises augmenting the amount of HNF1α protein in the tumor cell by administering the cell with a HNF1α protein.

For that purpose, the HNF1α protein may be chemically or enzymatically modified to improve stability or bioavailability.

### Pharmaceutical compositions:

The present invention further encompasses preparing pharmaceutical compositions useful for the above therapeutic methods. Examples of pharmaceutical formulations are provided hereafter. They can be readily adapted to the preparation of any type of pharmaceutical composition, comprising an active ingredient selected from HNF1α encoding nucleic acid, and HNF1α protein.

A preferred subject of the present invention is a pharmaceutical composition containing at least a HNF1α nucleotide sequence as defined above, preferably inserted in a recombinant virus.

The form of the pharmaceutical compositions, the route of administration, the dosage and the regimen naturally depend upon the condition to be treated, the severity of the illness, the age, weight, and sex of the patient, etc.

The pharmaceutical compositions of the invention can be formulated for a topical, oral, parenteral, intranasal, intravenous, intramuscular, subcutaneous or intraocular administration and the like.

Preferably, the pharmaceutical compositions contain vehicles which are pharmaceutically acceptable for a formulation capable of being injected, optionally directly into the tumour to be treated. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses of nucleic acids (sequence or vector) used for the administration can be adapted as a function of various parameters, and in particular as a function of the mode of administration used, of the relevant pathology, of the nucleic acid to be expressed, or alternatively of the desired duration of treatment. Generally, with regard to recombinant viruses, the latter are formulated and administered in the form of doses of between 104 and 1014 pfu/ml, and preferably 106 to 1010 pfu/ml. The term pfu ("plaque forming unit") corresponds to the infectivity of a virus solution, and is determined by infecting an appropriate cell culture and measuring, generally after 48 hours, the number of plaques of infected cells. The techniques for determining the pfu titer of a viral solution are well documented in the literature.

To prepare pharmaceutical compositions for protein therapy, an effective amount of the HNF1α protein may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

A HNF1α protein can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

In terms of using peptide or protein therapeutics as active ingredients, U.S. patents 4,608,251 ; 4,601,903 ; 4,599,231 ; 4,599,230 ; 4,596,792 and 4,572,770 provide useful information.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The active HNF1α protein may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds formulated for parenteral administration, such as intravenous or intramuscular injection, other pharmaceutically acceptable forms include, *e.g.* tablets or other solids for oral administration ; liposomal formulations ; time release capsules ; and any other form currently used, including cremes.

Other routes of administration are contemplated, including nasal solutions or sprays, aerosols or inhalants, or vaginal or rectal suppositories and pessaries.

In certain embodiments, the use of liposomes and/or nanoparticles is contemplated for the introduction of HNF1α protein into host cells. The formation and use of liposomes is generally known to those of skill in the art, and is also described below, as well as in the "gene therapy" section.

Nanocapsules can generally entrap compounds in a stable and reproducible way. To avoid side effects due to intracellular polmeric overloading, such ultrafine particles (sized around 0.1 µm) should be designed using polymers able to be degraded in vivo. Biodegradable polyalkyl-cyanoacrylate nanoparticles that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core.

The following information may also be utilized in generating liposomal formulations. Phospholipids can form a variety of structures other than liposomes when dispersed in water, depending on the molar ratio of lipid to water. At low ratios the liposome is the preferred structure. The physical characteristics of liposomes depend on pH, ionic strength and the presence of divalent cations. Liposomes can show low permeability to ionic and polar substances, but at elevated temperatures undergo a phase transition which markedly alters their permeability. The phase transition involves a change from a closely packed, ordered structure, known as the gel state, to a loosely packed, less-ordered structure, known as the fluid state. This occurs as a characteristic phase-transition temperature and results in an increase in permeability to ions, sugars and drugs.

Liposomes interact with cells via four different mechanisms : endocytosis by phagocytic cells of the reticuloendothelial system such as macrophages and neutrophils ; adsorption to the cell surface, either by non-specific weak hydrophobic or electrostatic forces, or by specific interactions with cell-surface components ; fusion with the plasma cell membrane by insertion of the lipid bilayer of the liposome into the plasma membrane, with simultaneous release of liposomal contents into the cytoplasm ; and by transfer of liposomal lipids to cellular or subcellular membrane, or vice versa, without any association of the liposome contents. Varying the liposome formulation can alter which mechanism is operative, although more than one may operate at the same time.

### DNA toxicity by estroprogestative

A further aspect of the invention provides a method for determining the toxicity of an estroprogestative agent on genomic DNA.

This method is preferably performed *in vitro,* in a acellular or cellular system. In these two systems the principle is to detect lesions in the HNF1α gene, either directly (*e.g.* by detecting an abnormal expression of the protein or an abnormal level of mRNA resulting from such mutations).

In the acellular system, control may be performed with a DNA sequence that encodes HNF1α but is not treated with the estroprogestative agent, and by a DNA sequence different from the HNF1α sequence and that does not exhibit a sequence targeted by estrogens or estroprogestatives.

In the cellular system, control may be performed using different cell lines expressing or not estrogen receptors and the HNF1α protein. To increase sensibility of the method, combination of cell lines deficient in mismatch repair can be used.

In a particular embodiment this method comprises
a) contacting an estroprogestative agent to be tested with a DNA sequence that encodes HNF1α (acellular system) or with a cell expressing HNF1α (cellular system);
b) determining the effect of the estroprogestative agent on the DNA sequence that encodes HNF1α or on the expression or activity of HNF1α;
wherein the presence of mutations in the HNF1α DNA sequence or an abnormal expression or activity of HNF1α in comparison respectively with a control DNA or cell is indicative of a toxic effect of the oestroprogestative agent on genomic DNA.

In another embodiment DNA adducts induced by estroprogestative can be searched at the HNF1α sequence, more specifically in the sequence around nucleotide 617. This system can employ a classical technique to search for DNA adducts using as a target, synthetic oligonucleotides identical to HNF1α region extending from nucleotides 600 to 640 transfected in cells expressing or not HNF1α.

Estroprogestative agents include, without limitation, 17beta-estradiol (E(2)), estrone (E(1)), diethylstilbestrol (DES), trans-dienestrol (alpha-DIES), cis-dienestrol (beta-DIES) catechol estrogens, 2-hydroxyestradiol (2-OH E2), or 4-hydroxyestradiol (4-OH E2), as well as any estroprogestative agent that could be identified in the future. This method is thus particularly useful as a tool in preclinical tests and to develop new estroprogestative agents substantially devoid of side effects.

In particular it is well-known that accumulation of premutagenic DNA lesions is implicated in the etiology of many degenerative diseases, aging and cancer. The present invention, by providing a new and reliable way of determining the toxicity of an estroprogestative exposure on DNA, is especially useful to screen estroprogestatives for their potential effect on tumorigenesis (Coumoul and Barouki, 2002).

The below examples and associated figures illustrate the invention without limiting its scope.

### LEGENDS TO THE FIGURES:

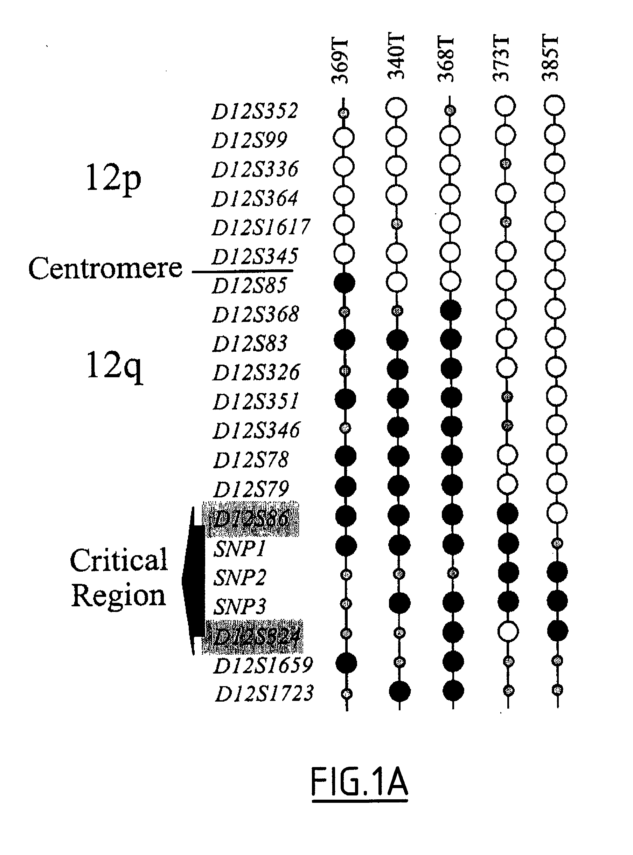

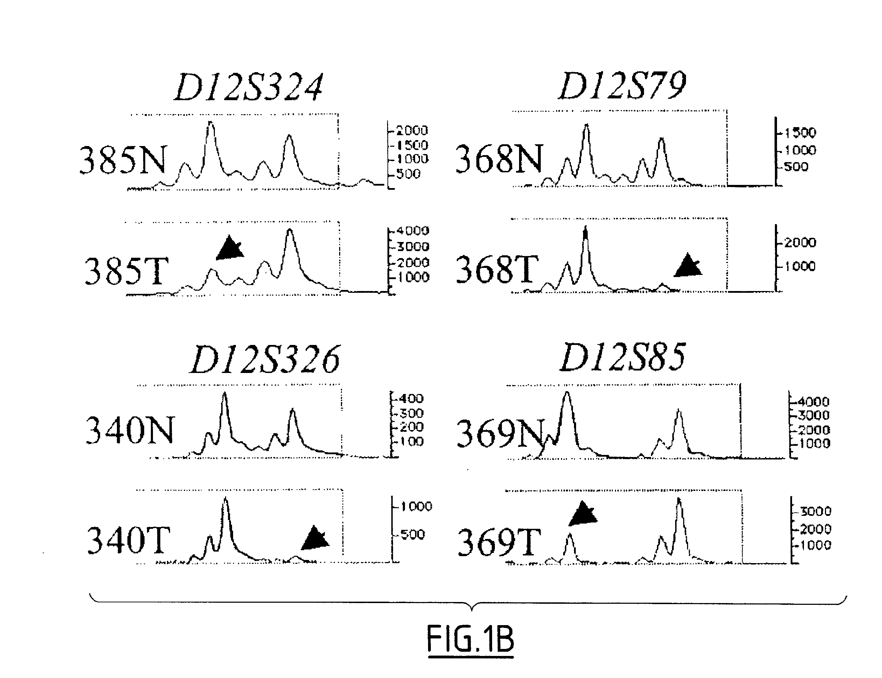

Figures 1A and 1B show the LOH at chromosome 12q in adenoma tumors. **Figure 1A:** Summary of LOH patterns in 5 adenoma tumors. Markers are shown along chromosome 12 according to the UCSC goldenpath sequencing map. Each vertical line represents a tumor sample. Black, white and gray circles represent LOH, retention of heterozygosity and non-polymorphic genotyping results, respectively. SNP 1 to 3 are polymorphic markers located in the TCF1 gene i.e. L17L (rs1169289), I27L (rs1169288) and IVS9-24, respectively. The critical region defined by genotyping is indicated on the left side between D12S86 and D12S324. **Figure 1B** show representative fluorescent electropherograms for LOH. Tumor and marker ID numbers are indicated. Fluorescent intensity scales are indicated on the right. Alleles lost are shown by an arrow.

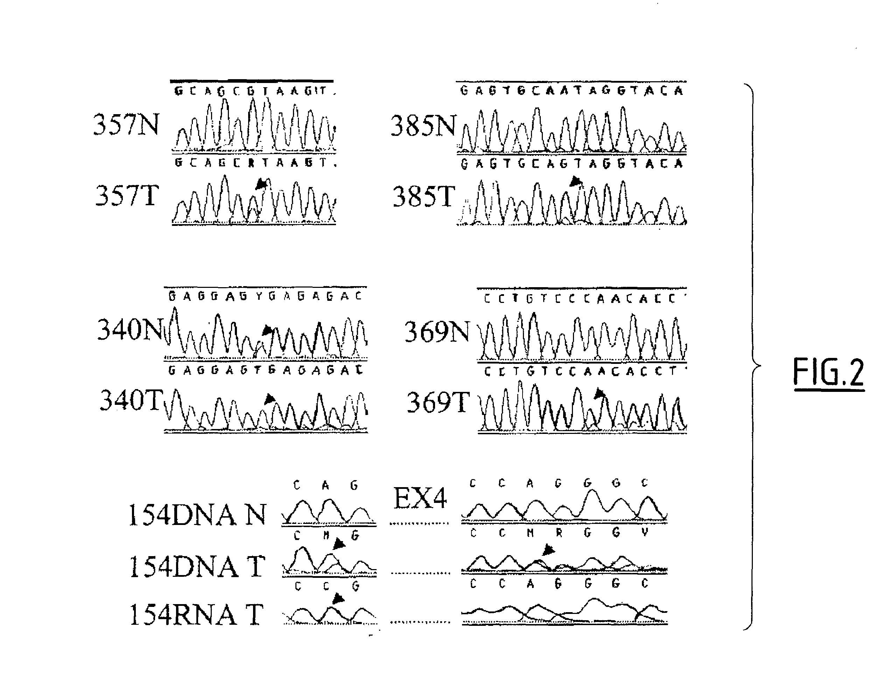

**Figure 2** shows representative TCF1 sequence variants. TCF1 sequence variants in adenoma cases (T) and their matching non-tumor DNA (N). Mutated bases are indicated by an arrow. In case 154, the RNA extraction was performed on the same tissue sample than the DNA extraction. In the non-tumor tissue 154 (154DNA N) no mutation was observed at nucleotide positions 749 and 872. At the tumoral DNA level (154DNA T) both mutated alleles were found on the same exon 4 PCR product whereas at the tumoral RNA level (154RNA T) only the 749A>C mutated allele is observed, the frameshifted mRNA is destabilized.

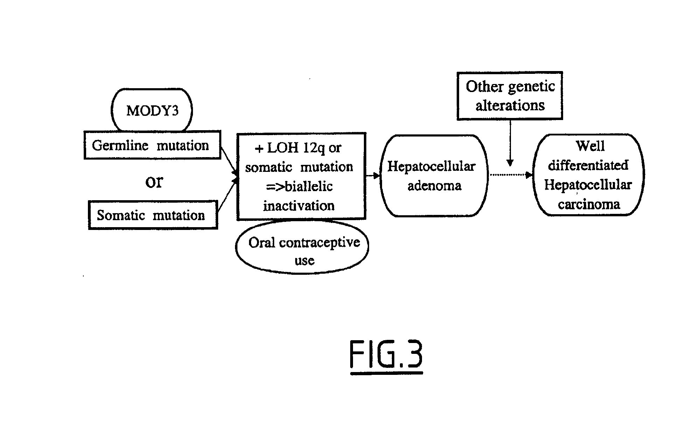

**Figure 3** is a schema of the occurrence of genetic alteration in liver tumors

### EXAMPLES:

### Example 1: Bi-allelic inactivation of HNF1α in hepatic tumors

### Methods

Patient samples. A series of 16 adenomas and of 30 HCC were collected in the Liver Tumor Database: Hôpital Pellegrin Bordeaux (28 cases), and Hôpital Antoine Béclère Clamart (2 cases). Tumor and non-tumor matching liver tissues were frozen immediately in liquid nitrogen and stored at -80°C until DNA extraction. In two additional cases (CHC361 and 372) only non-tumor DNAs were available. All the pathological slides and medical records were reviewed to confirm the diagnosis and the risk factors.

Genotyping. DNAs was extracted using a salting out procedure (Miller S.A. et al, (1988)). Allelic losses were determined by genotyping 400 markers from the LMS2 microsatellites panel (PE Biosystems) on tumor and corresponding non-tumor DNA as described previously (Laurent-Puig P. et al, (2001)). All physical map information used in this work is available in the Human Genome Project Working Draft at UCSC (http://genome.ucsc.edu/, (Lander E.S. et al (2001)).

Mutation analysis. HNF1a exons were amplified by PCR and purified with a Millipore PCR purification kit. Direct sequencing was performed on purified PCR products using big dye terminator chemistry (Applied Biosystems) on an Applied Biosystems A377 or 3700 sequencer. Detailed PCR protocols and sequence of the primers are described below. Analyses were performed using Factura and Autoassembler softwares (PE biosystems). The somatic origin of mutations was confirmed by sequencing DNA derived from the non-tumor corresponding tissue or from peripheral blood.

RT-PCR procedure. Tumor RNAs were extracted using Rneasy kit (Qiagen). Reverse-transcriptase PCRs were performed using Omniscript kit (Qiagen) with oligo-dT primers and AmpliTaq Perkin-Elmer polymerase to search for genetic alterations in adenomas and HCC tumors. In each case, the coding sequence from codon 12 to 625 was amplified in two overlapping fragments using primers located in exon 1 (GCCAGCTGCAGACGGAGC ; SEQ ID No. 5)) and 6 (GGCTCACCAGGCCCGATGG ; SEQ ID No 6)) and using primer from exon 2 (CCAGTCCCACCTGTCCCA ; SEQ ID No 7)) and 10 (CTGGGAGGAAGAGGCCATC ; SEQ ID No 8)). Subsequently, amplified fragments were directly sequenced with primers used for the amplification and internal primers.

### PCR protocols

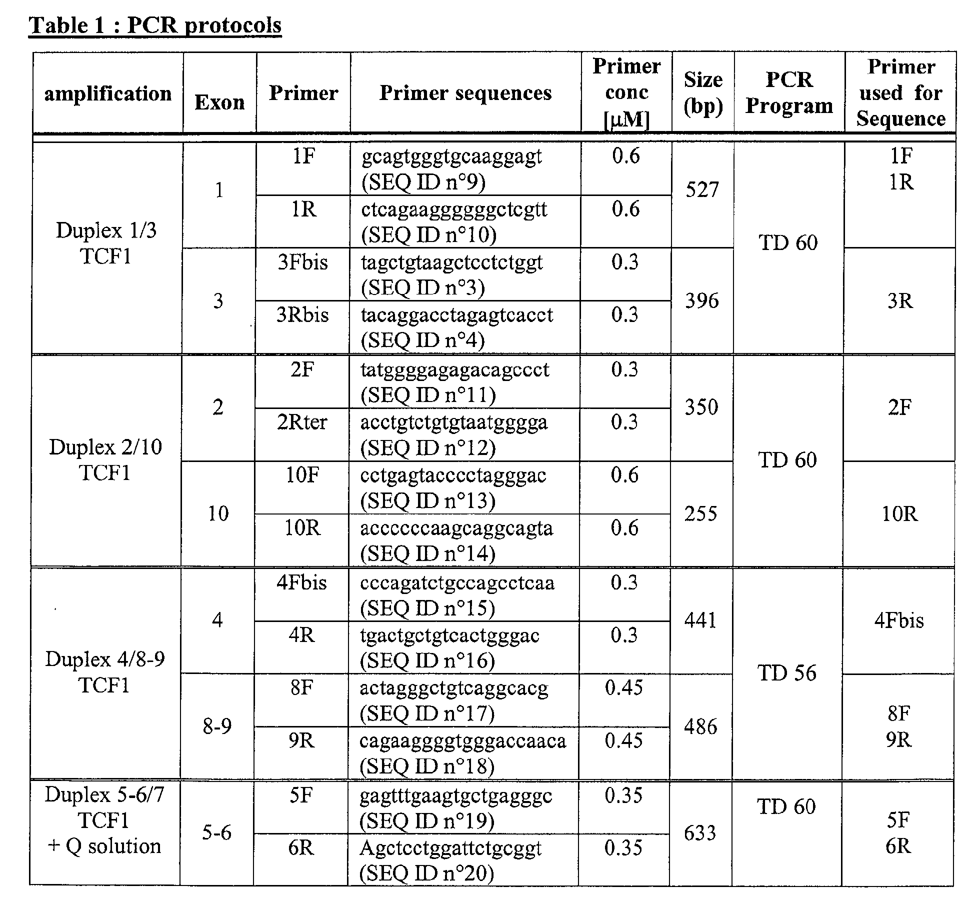

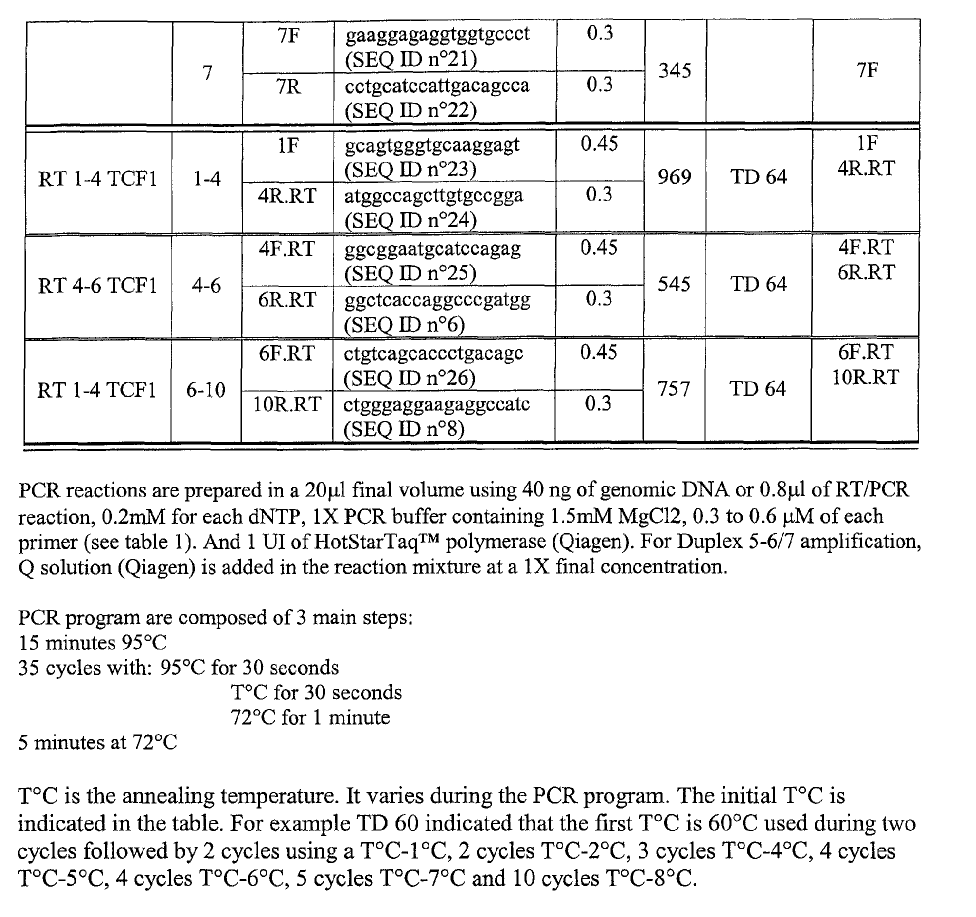

### Results

In a search for putative tumor suppressor genes, DNA from a series of 10 adenomas (solitary or multiple) and matched non-tumor tissue were genotyped for 400 microsatellite markers distributed over the genome. Chromosome 12q loss was observed in 5 of the tumor samples (Fig. 1A and 1B) with an 8 Mb minimal common region of deletion at 12q24, bounded by D12S86 and D12S324. Subsequent in silico analysis of this critical region revealed the presence of forty genes. The TCF1 gene, commonly known as HNF1α, was selected as a first candidate gene because it encodes a transcription factor, HNF1α, that is implicated in hepatocyte differentiation and that is required for liver-specific expression of several genes. Sequencing of the HNF1α transcripts after RT-PCR, and of all exons with their intron-boundaries, in DNA from 16 adenomas and the corresponding non-tumor tissue, was performed. The analysis of ten known single nucleotide polymorphisms (SNP)3 in tumor and non-tumor DNA for 14 of the cases confirmed the 12q24 deletion previously detected by microsatellite genotyping (Fig. 1A and 1B) and did not reveal additional microdeletion. In ten tumors, bi-allelic alteration of HNF1a was observed either by mutation and gene deletion in half of the tumors, or by the existence of two mutations in the remaining tumors (Table 2). In those tumors, a bi-allelic origin was confirmed by allele specific amplification and sequencing of RT-PCR products. In patient 340, one of the HNF1a alterations (R229X) was germline, similar to those found in patients with a MODY3 diagnosis (Kaisaki P.J. et al (1997)). Indeed, this patient presented a typical familial MODY phenotype and his father, who carried the same mutation, was also affected by hepatic adenomatosis. In the tumor sample of patient 340 the wild type allele was lost by a 12q deletion, while the mutant allele was retained, in accordance with the 2 hit model of tumor predisposition. For the remaining 9 adenomas, both HNF1α alterations were of somatic origin (including 4 with 12q LOH). Two of the patients (358 and 371) had the same somatic mutation (W206L) on one allele.

The inventors also screened 30 HCC samples and found bi-allelic mutations of HNF1 in two of them (Table 2). These mutations were not present in corresponding non-neoplastic liver tissues and all were point mutations rather than LOH. The two HNF1a mutated HCC had an infrequent presentation, with a very well differentiated tumor (Edmondson grade I) occurring in normal liver, and with no risk factor usually associated with HCC, such as infection by hepatitis B or C virus, or alcohol intake (Tsukuma H. et al. (1993)). These clinical observations are compatible with the hypothesis that these HCC arose in the cells of an adenomatous tumor, itself carrying inactivating mutations in HNF1α.

Among the 19 mutations observed in adenomas and HCC tumors, 9 were non-sense or frameshift mutations leading to a premature stop codon (Table 2). The 10 remaining mutations all occurred in the DNA binding domain, including two that were in the region required for specific HNF binding and 8 in the homeodomain. All these missense mutations occurred at amino acids that are highly conserved in both amphibians and rodents, suggesting that they are important for protein function. These mutations were not found in more than 500 alleles screened in the literature (Ellard S. et al (2000)).

Two insertions of a cytosine and one 13 base pair deletion were observed in the cytosine tract (C8-position 872), but these three tumors did not exhibit a microsatellite instability phenotype as defined by the National Cancer Institute Workshop on Microsatellite Instability for cancer detection (Boland C.R. et al (1998)). Interestingly, the mutations occurring at this hotspot resulted in complete (CHC154) or partial (CHC370 and 358) destabilization of the frameshifted mRNA, as was shown by the dramatic decrease in the intensity of the peaks corresponding to the frameshifted mRNA (Fig. 2). This frameshift mutation, which leads to the truncated protein P291fsinsC, has been observed previously in 20% of patients with MODY3 and overexpression of this mRNA in transfection experiments had a dominant negative effect on promoters normally induced by HNF1α (Vaxillaire M. et al. (1999), Yamagata K. et al. (1998)). Nevertheless, HNF1α activity will be absent in the adenomas due to the destabilization of this mRNA and the mutation of the second allele. As the 19 mutations described here led to either protein truncation, modification of the DNA-binding domain and/or RNA destabilization, these mutations are likely to generate a decrease in HNF1α transactivation activity (Vaxillaire M. et al. (1999)). Furthermore, in all the mutated adenoma and carcinoma samples, the alterations were bi-allelic and expected to result in a dramatic loss of activity.

The inventors also found germline HNF1a mutations in two additional patients who had previously a liver tumor resection in a context of familial diabetes. Patient 361 presented a HCC developed in an adenoma, while patient 372 had a hyperplasic liver tumor (Table 2). The G574S mutation was recently described with a high prevalence in African diabetics (Collet C. et al. (2002)), whereas, the second germline mutation, R583Q, altered a highly conserved amino-acid that has not been observed previously. These results suggest a role of germline HNF1α mutation in predisposition to liver benign tumor development and may explain a previous observation describing cosegregation of liver adenoma with diabetes mellitus in a large family (Foster J.H. et al. (1978)). Because most of the HNF1α mutations in MODY3 patients and in tumors are unique, further studies are required to elucidate the relationship between genotype and phenotype.

Finally, 6 adenomas showed neither 12q LOH nor HNF1α mutations. These cases could be due to undetected alteration in the non-coding region or in the promoter of HNF1α that could have been missed by our screening process. Alternatively, mutations in another gene could play a part in the occurrence of adenomas in those patients such as the other transcription factors mutated in rare subtypes of MODY (HNF1β, HNF4α and IPF-1). However, the present results did not indicate another common putative tumor suppressor gene, as there were no other recurrently deleted chromosome regions.

In these series, HNF1α alteration is the only genetic event detected in most of the adenoma cases but it is associated with multiple genetic alterations in HCC. These results suggest that acquisition of a malignant phenotype by the tumor requires accumulation of additional genetic alterations such as multiple LOH or TP53 mutations (Table 2). The observation of HNF1α bi-allelic alteration in human liver tumors meets the criteria of the classical two-hit recessive model of oncogenesis (Knudson A.G. et al. (1971), Comings D.E. (1973)) and supports the hypothesis that HNF1α is a tumor suppressor gene altered early in carcinogenesis leading to adenoma formation. The hypothesis of a tumor suppressor function for HNF1α is also reinforced by the phenotypes observed in the two models of homozygous HNF1α deficient mice. These mice have either a dramatic liver enlargement caused by an increased proliferation of hepatocytes (Pontoglio M. et al. (1996)) or a liver enlargement with central lobular hypertrophy and degeneration of individual hepatocytes (Lee Y.H. et al. (1998)). Interestingly in the present series, mutated adenomas appeared to have typical clinical presentations, *i.e.* most of the cases occurred in women with mild or long term use of oral contraceptives suggesting a link between HNFα alteration, cellular proliferation and the use of estrogen/progestin.

This study demonstrates that HNF1α inactivation is an early oncogenic event in liver adenomas and in some hepatocellular carcinomas, and that inactivation occurs sporadically or in association with familial germline mutation (Fig. 3). These results have immediate clinical implications. In particular, MODY3 patients should benefit from liver monitoring to prevent and detect early tumor occurrence, and patients presenting liver adenoma, especially in a familial context, should be investigated for diabetes.

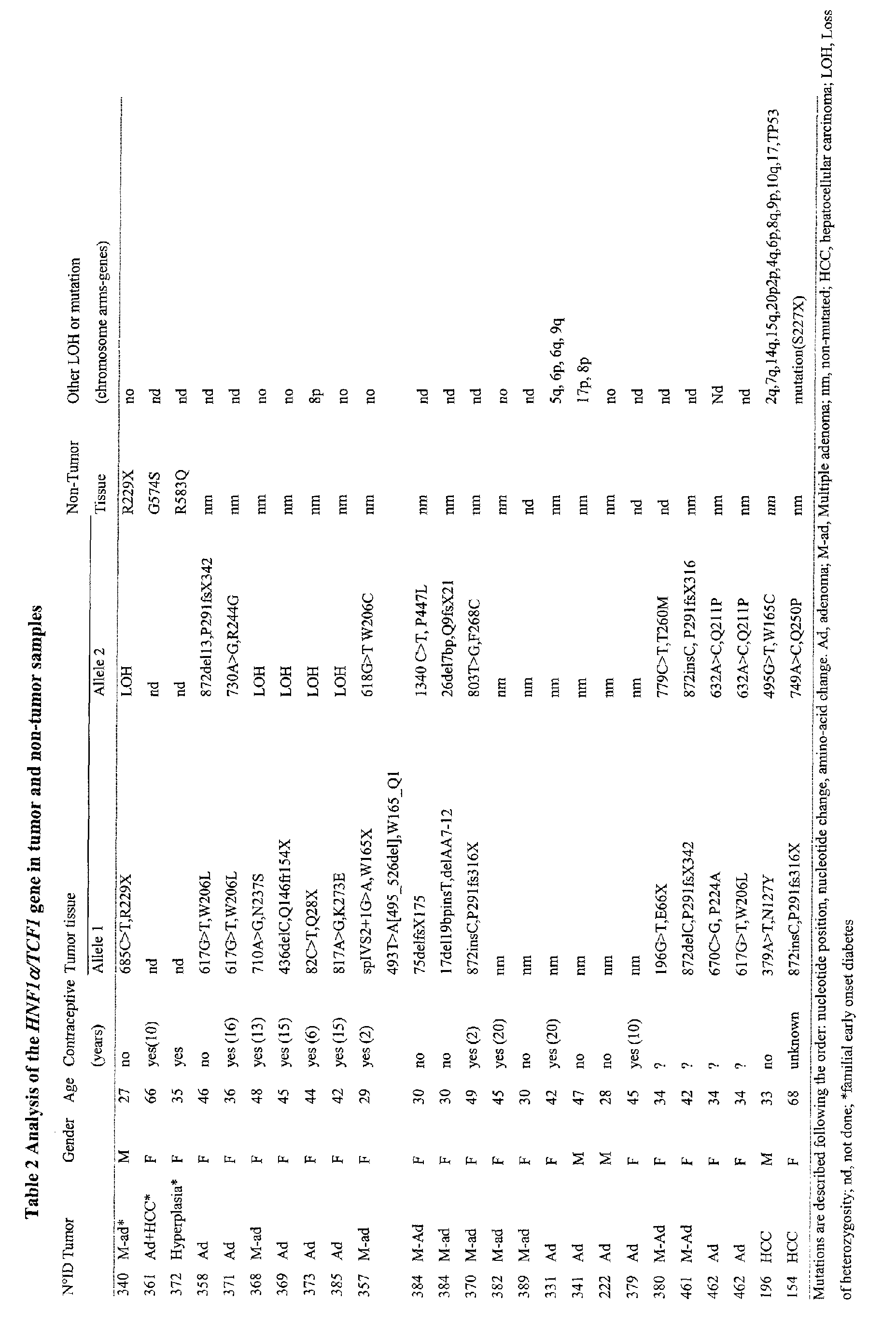

### Example 2: Mutations in HNF1α in colon cancer

### Material and methods :

### Patients and methods

In a primary screening, a series of 10 adenomas and 29 colorectal cancers were collected in two French pathological departments: Hôpital Européen Georges Pompidou (29 cases), and CHU Dijon (10 cases). Tumor and non-tumor matching tissues were frozen immediately in liquid nitrogen and stored at -80°C until DNA extraction. All tumors were characterized by five microsatellites (BAT26, BAT25, D2S123, D5S346 and D17S250) and the MSI-H phenotype was assigned following the criteria defined by international consensus conference on microsatellite instability (Tsukuma, H. et al. (1993)). Twelve tumors were identified MSI-H, 17 MSS and all adenomas were MSS. In a second screening, 52 MSI-H colorectal cancers were selected for HNF1α analysis in the Department of Genetics, Cliniques Universitaires St Luc , Brussels , Belgium.

### Mutation analysis

HNF1α mutations were screened by direct sequencing of all exons with their intronic boundaries, as described in example 1. Sequence analyses were performed using Factura and Autoassembler softwares (Applied Biosystems Courtaboeuf, France). The somatic origin of mutations was confirmed by sequencing matched non-tumoral DNA.

### RT-PCR

In the first series, nineteen colorectal tumors were available for RNA extraction using Rneasy kit (Qiagen). Reverse-transcriptase PCRs were performed using Omniscript kit (Qiagen) with oligo-dT primers and AmpliTaq Perkin-Elmer polymerase to search for genetic alterations in colorectal cancer tumors. In each case, the coding sequence from codon 12 to 625 was amplified in two overlapping fragments using primers located in exon 1 (GCCAGCTGCAGACGGAGC ; SEQ ID n° 5) and 6 (GGCTCACCAGGCCCGATGG ; SEQ ID n° 6) and using primer from exon 2 (CCAGTCCCACCTGTCCCA ; SEQ ID n° 7) and 10 (CTGGGAGGAAGAGGCCATC ; SEQ ID n° 8). Subsequently, amplified fragments were directly sequenced with primers used for the amplification and with internal primers.

### Results

Colorectal cancer is characterized by the existence of different entities known as microsatellite stable (MSS) or unstable tumors (MSI-H). At first, a series of 10 adenomas and 29 colon cancers regardless of the MSI status were screened for HNF1α mutations on the entire coding sequence. Three mutations in MSI-H tumors were found in the exon 4 poly-cytosin (C)₈ or (C)₉ tract and consisted of a cytosin deletion at position 291. To further characterize the importance of HNF1 mutations in the subgroup of MSI-H tumors, 52 MSI-H additional samples were screened for exon 4 alterations and 23% CI_{95%}[14%-36%] of MSI-H tumors were found to harbor frameshifts at the cytosin track. The (C)₉ allele was significantly more frequently mutated than the (C)₈ allele (22% versus 8% p=0.03) demonstrating a higher instability of the longer repetition. This shows a role for HNF1 in MSI-H colorectal carcinogenesis.

Results of these experiments are given in greater detail herebelow.

### Screening for HNF1α mutation in colorectal adenoma and carcinoma :

Sequencing all HNF1α exons with their intron-boundaries was performed in 10 colon adenomas and 29 colorectal carcinomas DNAs. A one base pair deletion in exon 4 was found in 3 colorectal carcinomas. In all three cases it occurred at nucleotide 872 within a polymorphic poly-cytosin (C)₈ or (C)₉ tract. These mutations led to a frameshift and a protein truncation at position 342. All three HNF1α mutated tumors exhibited a MSI-H phenotype. 19 colorectal cancer samples, including the three mutated cases, were subjected to RNA mutation screening on 95% of the coding sequence Screening at RNA level did not reveal additional mutation in Adenoma or MSS tumors. Although very weakly expressed in one case, the RNA corresponding to the 872del C allele could be detected suggesting the absence of a destabilizing effect of this mutation.

### Prevalence of the 872delC mutation in MSI-H colorectal carcinoma

Fifty-two MSI-H colorectal DNA samples were further sequenced in exon 4 in order to evaluate the actual frequency of the polyC tract alteration in this subgroup of tumors. Twelve additional MSI-H colorectal tumors were found mutated leading to a mutation rate of 23% IC_{95%} [14%-36%] in MSI-H colorectal cancers. All but one mutation consisted of a deletion of one cytosin leading to a stop codon at position 342. The remaining case consisted of an insertion of one cytosin leading to premature stop codon at position 316.

### Mutations occurred more frequently on the (C)₉ allele :

The nucleotide 864 (G>C) of HNF1α is polymorphic in the general population generating a (C)₈ or (C)₉ polyC tract. The (C)₈ and (C)₉ alleles were observed at a frequency of 72% and 28% in the colorectal cancer patients. No significant difference in the frequency of the different genotypes distribution was observed between MSI-H and MSS tumors. However, in the MSI-H tumors, the (C)₉ allele was significantly more frequently mutated than the allele (C)₈ 22% versus 8% p=0.03. Finally, in the group of MSI-H patients, the clinical and anatomopathological characteristics of the HNF1α mutated tumors did not differ from those non-mutated.

The frameshift mutations found in HNF1α exon 4 are predicted to lead to the production of truncated proteins containing the dimerization and the DNA binding domain. In contrast these truncated proteins lack the COOH-terminal part containing the transactivation domain. In MODY3 patients, the most common HNF1α mutation (20%) is an insertion of a cytosine in the exon 4 polyC tract leading to the synthesis of a protein of 315 amioacids. Over expression of this truncated protein in MIN6 and C33 cells revealed the dominant negative function of the P291fsinsC mutation (Schott, et al. (1997)); (Vaxillaire, et al. (1999)). It could also be the case for the 872delC observed in colorectal cancers, which would contrast with the biallelic HNF1α inactivation observed in hepatocellular adenoma. However in liver adenoma a variety of mutations exist and exon 4 frameshift mutation encounts for only 15% of them. Moreover, in hepatocellular adenoma exon 4 frameshift mutation leads to RNA destabilization. In MSI-H colorectal cancer no destabilization of the frameshift RNA was observed and mutations seemed to be mono-allelic. These two arguments are in favor of a dominant negative effect of the P291fsdelC mutation in the intestine origin cells.

The HNF1α polyC tract in exon 4 is polymorphic in the Caucasian population. A higher rate of mutations occurring on the C₍₉₎ allele in comparison with the average frequency suggested a preferential targeting of the longer tract by mismatch repair deficiency. According to this observation, this polymorphism could be implied in the risk of colorectal cancer in general population.

### References

- Baumhueter, S. et al. HNF-1 shares three sequence motifs with the POU domain proteins and is identical to LF-B1 and APF. Genes Dev 4, 372-9. (1990).
- Boland, C.R. et al. A National Cancer Institute Workshop on Microsatellite Instability for cancer detection and familial predisposition: development of international criteria for the determination of microsatellite instability in colorectal cancer. Cancer Res 58, 5248-57. (1998).
- Boland, C. R., Thibodeau, S. N., Hamilton, S. R., Sidransky, D., Eshleman, J. R., Burt, R. W., Meltzer, S. J., Rodriguez-Bigas, M. A., Fodde, R., Ranzani, G. N., and Srivastava, S. A National Cancer Institute Workshop on Microsatellite Instability for cancer detection and familial predisposition: development of international criteria for the determination of microsatellite instability in colorectal cancer, Cancer Res. *58:* 5248-57., 1998.
- Cereghini, S., Yaniv, M. & Cortese, R. Hepatocyte dedifferentiation and extinction is accompanied by a block in the synthesis of mRNA coding for the transcription factor HNF1/LFB1. Embo J 9, 2257-63. (1990).
- Chiche, L. et al. Liver adenomatosis: reappraisal, diagnosis, and surgical management: eight new cases and review of the literature. Ann Surg 231, 74-81. (2000).
- Chouard et al, 1997, Biochimie, 79 : 707-715.
- Chouard, T. et al. A distal dimerization domain is essential for DNA-binding by the atypical HNF1 homeodomain. Nucleic Acids Res 18, 5853-63. (1990).
- Collet, C. et al. Prevalence of the missense mutation Gly574Ser in the hepatocyte nuclear factor-1alpha in Africans with diabetes. Diabetes Metab 28, 39-44. (2002).
- Comings, D.E. A general theory of carcinogenesis. Proc Natl Acad Sci U S A 70, 3324-8. (1973).
- Coumoul and Barouki, Medecine/Sciences 2002, 18:86.
- Edmondson, H.A., Henderson, B. & Benton, B. Liver-cell adenomas associated with use of oral contraceptives. N Engl J Med 294, 470-2. (1976).
- Ellard, S. Hepatocyte nuclear factor 1 alpha (HNF-1 alpha) mutations in maturity- onset diabetes of the young. Hum Mutat 16, 377-85. (2000).
- Ferrell, L.D. Hepatocellular carcinoma arising in a focus of multilobular adenoma. A case report. Am J Surg Pathol 17, 525-9. (1993).
- Flejou, J.F. et al. Liver adenomatosis. An entity distinct from liver adenoma? Gastroenterology 89, 1132-8. (1985).
- Foster, J.H., Donohue, T.A. & Berman, M.M. Familial liver-cell adenomas and diabetes mellitus. N Engl J Med 299, 239-41. (1978).
- Frain, M. et al. The liver-specific transcription factor LF-B1 contains a highly diverged homeobox DNA binding domain. Cell 59, 145-57. (1989).
- Goldfarb, S. Sex hormones and hepatic neoplasia. Cancer Res 36, 2584-8. (1976).
- Harlow et al, 1988 editors, Antibodies : A laboratory manual
- Howell, R.R., Stevenson, R.E., Ben-Menachem, Y., Phyliky, R.L. & Berry, D.H. Hepatic adenomata with type 1 glycogen storage disease. Jama 236, 1481-4. (1976).
- Kaisaki, P.J. et al. Mutations in the hepatocyte nuclear factor-1alpha gene in MODY and early-onset NIDDM: evidence for a mutational hotspot in exon 4. Diabetes 46, 528-35. (1997).
- Knudson, A.G., Jr. Mutation and cancer: statistical study of retinoblastoma. Proc Natl Acad Sci U S A 68, 820-3. (1971).
- Lander, E.S. et al. Initial sequencing and analysis of the human genome. Nature 409, 860-921. (2001).
- Laurent-Puig, P. et al. Genetic alterations associated with hepatocellular carcinomas define distinct pathways of hepatocarcinogenesis. Gastroenterology 120, 1763-73. (2001).
- Laurent-Puig, P., Legoix, P., Bluteau, O., Belghiti, J., Franco, D., Binot, F., Monges, G., Thomas, G., Bioulac-Sage, P., and Zucman-Rossi, J. Genetic alterations associated with hepatocellular carcinomas define distinct pathways of hepatocarcinogenesis, Gastroenterology. *120:* 1763-73., 2001.
- Lee, Y.H., Sauer, B. & Gonzalez, F.J. Laron dwarfism and non-insulin-dependent diabetes mellitus in the Hnf- 1alpha knockout mouse. Mol Cell Biol 18, 3059-68. (1998).
- Lengauer, C., Kinzler, K. W., and Vogelstein, B. Genetic instability in colorectal cancers, Nature. *386:* 623-7., 1997.
- Miller, S.A., Dykes, D.D. & Polesky, H.F. A simple salting out procedure for extracting DNA from human nucleated cells. Nucleic Acids Res 16, 1215. (1988).
- Nicosia, A. et al. A myosin-like dimerization helix and an extra-large homeodomain are essential elements of the tripartite DNA binding structure of LFB1. Cell 61, 1225-36. (1990).
- Pontoglio, M. et al. Hepatocyte nuclear factor 1 inactivation results in hepatic dysfunction, phenylketonuria, and renal Fanconi syndrome. Cell 84, 575-85. (1996).
- Reeck et al, 1987, Cell, 50 : 667.
- Sale, G.E. & Lerner, K.G. Multiple tumors after androgen therapy. Arch Pathol Lab Med 101, 600-3. (1977).
- Sambrook et al, 1989, Molecular Cloning : A laboratory manual, second edition Cold Spring Harbor Laboratory press, Cold Spring Harbor, New-York.
- Schott, O., Billeter, M., Leiting, B., Wider, G. & Wuthrich, K. The NMR solution structure of the non-classical homeodomain from the rat liver LFB1/HNF1 transcription factor. J Mol Biol 267, 673-83. (1997).
- Tsukuma, H. et a1. Risk factors for hepatocellular carcinoma among patients with chronic liver disease. N Engl J Med 328, 1797-801. (1993).
- Vaxillaire, M. et al. Anatomy of a homeoprotein revealed by the analysis of human MODY3 mutations. J Biol Chem 274, 35639-46. (1999).
- Yamagata, K. et al. Mutation P291fsinsC in the transcription factor hepatocyte nuclear factor-1alpha is dominant negative. Diabetes 47, 1231-5. (1998).
- Yamagata, K. et al. Mutations in the hepatocyte nuclear factor-1alpha gene in maturity-onset diabetes of the young (MODY3). Nature 384, 455-8. (1996).

## Claims

1. An *in vitro* method for the diagnosis of a tumor in a patient, which method comprises determining the expression or activity of Hepatocyte Nuclear Factor 1α (HNF1α) in a biological sample, wherein an abnormal expression or activity in comparison with a control sample is indicative of a tumor or of a susceptibility to develop a tumor.

2. The method according to claim 1, wherein the step of determining the expression or activity of HNF1α comprises detecting a mutation in the HNF1α gene or in the promoter thereof, wherein said mutation results in impaired expression or activity of the HNF1α protein.

3. The method according to claim 2, wherein the mutation is bi-allelic.

4. The method according to any of claims 2 or 3, wherein the mutation is located in the coding sequence of the HNF1α gene.

5. The method according to any of claims 2 to 4, wherein the mutation results in full inactivation of HNF1α.

6. The method according to any of claims 2 to 5, wherein the mutation is detected by sequencing of the HNF1α gene.

7. The method according to any of claims 2 to 5, wherein the mutation is selected from the group consisting of:
685C>T;617G>T;710A>G;436delC;82C>T;817A>G;spIVS2+1G>A;
493T>A[495_526del]; 17dell 9bpinsT;872insC; 196G>T;872delC;
670C>G;379A>T;872insC;872del13;730A>G;618G>T;1340C>T;26del17bp;803T>G; 779C>T;872insC;632A>C;495G>T; and 749A>C.

8. The method according to claim 7, wherein the mutation is a transversion G>T at nucleotide 617 resulting in an aminoacid substitution W206L.

9. The method according to claim 1, wherein the step of determining the expression or activity of HNF1α comprises assessing the level of mRNA that encodes the HNF1α protein.

10. The method according to claim 1, wherein the step of determining the expression or activity of HNF1α comprises assessing the level of HNF1α protein or the level of activity thereof.

11. The method according to any of claims 1 to 10, wherein the tumor is a liver tumor.

12. The method according to claim 11, wherein the tumor is hepatic adenoma or hepatocellular carcinoma.

13. The method according to any of claims 1 to 12, wherein the patient is a Maturity-Onset Diabetes of the Young type 3 (MODY3) patient.

14. The method according to any of claims 1 to 13, wherein the patient is a female who uses estroprogestative for contraception.

15. The method according to any of claims 1 to 11, wherein the tumor is a colon tumor.

16. The method according to claim 1, which method comprises detecting a mutation in Hepatocyte Nuclear Factor 1α (HNF1α) gene in a liver sample, which mutation results in bi-allelic inactivation of HNF1α that is indicative of liver tumor selected from a hepatic adenoma and a hepatocellular carcinoma, or is indicative of a susceptibility to develop said liver tumor.

17. The method according to claim 1, which method comprises detecting a mutation in HNF1α gene in a colon sample, which mutation results in mono-allelic inactivation of HNF1α that is indicative of a colon tumor selected from a colorectal adenoma or carcinoma, of the MSI-H phenotype or is indicative of a susceptibility to develop said colon tumor.

18. A method for determining the susceptibility of a subject to develop a colon tumor, which method comprises detecting a polymorphism consisting of a 864G>C transversion in the HNF1α gene, wherein said transversion is correlated with a higher risk to develop a colon tumor.

19. Use of (i) a nucleic acid that encodes a functional HNF1α protein or (ii) a functional HNF1α protein, for the preparation of a medicament for the prevention or treatment of a tumor.

20. The use according to claim 19, wherein the tumor is a cancer.

21. The use according to any of claims 19 or 20, wherein the tumor is a liver or a colon tumor.

22. A method for determining the toxicity of an estroprogestative agent on genomic DNA, which method comprises
a) contacting an estroprogestative agent to be tested with a DNA sequence that encodes HNF1α or with a cell expressing HNF1α;
b) determining the effect of the estroprogestative agent on the DNA sequence that encodes HNF1α or on the expression or activity of HNF1α;
wherein the presence of mutations in the HNF1α DNA sequence or an abnormal expression or activity of HNF1α in comparison with an untreated control DNA or cell is indicative of a toxic effect of the estroprogestative agent on genomic DNA.
